(19) Europäisches Patentamt / European Patent Office / Office européen des brevets

(11) **EP 4 803 095 A2**

(12) **EUROPEAN PATENT APPLICATION**


(43) Date of publication:
**09.09.2026 Bulletin 2026/37**

(21) Application number: **26189848.0**

(22) Date of filing: **27.09.2019**

(51) International Patent Classification (IPC):
***A61K 38/26*** *(2006.01)*

(52) Cooperative Patent Classification (CPC):
**C07K 14/605; A61K 9/0019; A61K 38/26; A61K 47/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2018 EP 18197755**

(62) Document number(s) of the earlier application(s) in accordance with Art. 76 EPC:
**19773860.2 / 3 856 766**



(71) Applicant: **Zealand Pharma A/S**
**2860 Søborg (DK)**

(72) Inventors:
- **GIEHM, Lise**
**2860 Søborg (DK)**
- **MELANDER, Claes**
**2860 Søborg (DK)**
- **MØLLER, Eva Horn**
**2860 Søborg (DK)**

(74) Representative: **Mewburn Ellis LLP**
**Aurora Building**
**Counterslip**
**Bristol BS1 6BX (GB)**


Remarks:
This application was filed on 03-07-2026 as a divisional application to the application mentioned under INID code 62.

(54) **FORMULATIONS OF GLUCAGON-LIKE-PEPTIDE-2 (GLP-2) ANALOGUES**


(57) Liquid formulations of GLP-2 analogues that make them suitable for long term storage as liquids and/or that makes them especially suitable for delivery by a drug delivery device are described. Solid compositions comprising acetate salts of glucagon-like peptide 2 (GLP-2) analogues useful for making the liquid formulations are also described. The development of these liquid formulations is based on the finding that acetate present in the formulation that originates from the GLP-2 analogues has an effect on the viscosity of the formulation, that during long term storage at 2-8°C of GLP-2 analogues, the concentration dependence for covalent oligomer formation is inversely dependent on increasing concentration of the GLP-2 analogue, and that GLP-2 analogues used in the formulations are not compatible with phosphate buffer commonly used in the prior art to reconstituted powdered or lyophilized GLP-2 compositions.





Processed by Luminess, 75001 PARIS (FR)

## Description

### Field of the Invention

The present invention relates to formulations of glucagon-like-peptide-2 (GLP-2) analogues and their medical use, for example in the treatment and/or prevention of stomach and bowel-related disorders and for ameliorating side effects of chemotherapy and radiation therapy. Furthermore, solid compositions comprising acetate salts of glucagon-like peptide 2 (GLP-2) analogues useful for making the liquid formulations are also described.

### Background of the Invention

Human GLP-2 is a 33-amino-acid peptide with the following sequence: Hy-His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp-OH. It is derived from specific post-translational processing of proglucagon in the enteroendocrine L cells of the intestine and in specific regions of the brainstem. GLP-2 binds to a single G-protein-coupled receptor belonging to the class II glucagon secretin family.

GLP-2 has been reported to induce significant growth of the small intestinal mucosal epithelium via the stimulation of stem cell proliferation in the crypts, and by inhibition of apoptosis in the villi (Drucker et al., 1996, Proc. Natl. Acad. Sci. USA 93: 7911-7916). GLP-2 also has growth effects on the colon. Furthermore, GLP-2 inhibits gastric emptying and gastric acid secretion (Wojdemann et al., 1999, J. Clin. Endocrinol. Metab. 84: 2513-2517), enhances intestinal barrier function (Benjamin et al., 2000, Gut 47: 112-119), stimulates intestinal hexose transport via the upregulation of glucose transporters (Cheeseman, 1997, Am. J. Physiol. R1965-71), and increases intestinal blood flow (Guan et al., 2003, Gastroenterology, 125: 136-147).

It has been recognised in the art that glucagon-like peptide-2 receptor analogues have therapeutic potential for the treatment of intestinal diseases. However, the native hGLP-2, a 33 amino acid gastrointestinal peptide, is not a useful in a clinical setting due to its very short half-life in humans of around 7 minutes for full length GLP-2 [1-33] and 27 minutes for truncated GLP-2 [3-33]. In large part, the short half-life is due to degradation by the enzyme dipeptidylpeptidase IV (DPP-IV). Accordingly, there have been attempts in the art to develop GLP-2 receptor agonists with better pharmacokinetic characteristics, in particular to improve the half-life of GLP-2 molecules. By way of example, GLP-2 analogues with substitutions have been suggested such as e.g. GLP-2 analogues containing Gly substitution at position 2 ([hGly2] GLP-2, teduglutide) which increases the half-life from seven minutes (native GLP-2) to about two hours. Acylation of peptide drugs with fatty acid chains has also proven beneficial for prolonging systemic circulation as well as increasing enzymatic stability without disrupting biological potency. However, while these attempts have improved the pharmacokinetics of GLP-2 analogues, and they are sometimes described in the art as "long acting", it must be kept in mind that this is in comparison to native hGLP-2 with half-lives of the order of several hours, rather than minutes. This in turn means that the GLP-2 analogues still need to be administered to patients one or more times per day.

US 5,789,379 discloses GLP-2 analogues for administration by injection. The analogues were provided as powdered peptides and mixed with phosphate buffered saline (PBS) prior to injection at pH of 7.3-7.4 with a GLP-2 concentration of 130 mg/ml. In some instances, the GLP-2/PBS composition was mixed with gelatin to provide a depot formed from a solution of 130 mg/l GLP-2 in PBS/15% gelatin. US 5,789,379 does not disclose stable aqueous liquid formulations of GLP-2 analogues and the GLP-2 analogues are generally reconstituted from powder prior to injection.

In WO 97/39031 and US 6,184,201, the GLP-2 analogue, [Gly$^2$]GLP-2 is disclosed. Here the alanine in position 2 has been replaced with glycine to make the peptide resistant to DPP IV cleavage. As with US 5,789,379, the GLP-2 analogue was provided as a powdered peptide and mixed with saline, PBS or 5% dextrose prior to injection, optionally adding acetic acid as a solubility enhancer.

WO 02/066511 describes GLP-2 analogues having an extended half-life *in vivo* and their use as medicaments in the treatment of gastrointestinal disorders, such as inflammatory bowel diseases. The GLP-2 analogues were stored in lyophilized form and reconstituted for administration in media, for example using saline or PBS.

WO 01/41779 describes the use of h[Gly$^2$]GLP-2 as a pre-treatment for inhibiting chemotherapy induced apoptosis and promoting cell survival. The h[Gly$^2$]GLP-2 is delivered by subcutaneous or intravenous injection or infusion after reconstituting the analogue in PBS.

WO 2001/049314 is directed to formulations of GLP-2 peptides and analogues thereof exhibiting superior stability following storage and/or exposure to elevated temperatures. The GLP-2 compositions comprise a GLP-2 peptide or an analogue thereof, a phosphate buffer, L-histidine, and mannitol.

WO 2006/117565 describes GLP-2 analogues which comprise one of more substitutions as compared to [hGly$^2$] GLP-2 and which improved biological activity *in vivo* and/or improved chemical stability, e.g. as assessed in *in vitro* stability assays. In particular, GLP-2 analogues are described which have substitutions at one or more of positions 8, 16, 24 and/or 28 of the wild-type GLP-2 sequence, optionally in combination with further substitutions at position 2 and one or more of

positions 3, 5, 7, 10 and 11, and/or a deletion of one or more of amino acids 31 to 33. These substitutions may also be combined with the addition of a N-terminal or C-terminal stabilizing peptide sequence. The daily or twice daily administration of these GLP-2 analogues is also described. Among the molecules disclosed in WO 2006/117565 is glepaglutide (ZP1848) which has been designed to be stable in liquid formulations, and is typically administered by daily dosing using an injection pen.

It remains a problem in this area to improve the formulation of GLP-2 analogues, in particular to provide stable liquid formulations that are capable of long term storage without undue levels of physical or chemical degradation of the active monomeric form of the peptide occurring. In liquid formulations of peptide drugs, the chemical pathways that can operate include the formation of covalently linked dimers and oligomers of the peptide, reducing the amount of the active monomeric form of the peptide through the formation of these covalently linked high molecular weight oligomeric products. The law of mass action means that it is normally the case that the higher the concentration of a peptide drug in a formulation, the higher the probability of formation of covalently bonded oligomeric products.

It would also be a goal in the area of GLP-2 analogue formulation to provide formulations in which the viscosity of the formulation is controlled within a range that makes it suitable for use in delivery devices such as pre-filled syringes, infusion pumps, wearable injectors or auto-injectors.

## Summary of the Invention

Broadly, the present invention is based on studies reported in the examples that led to surprising findings relating to liquid formulations of GLP-2 analogues that make them suitable for long term storage as liquids and/or that makes them especially suitable for delivery by a drug delivery device.

In a first study, the inventors found that acetate present in the formulation that originates from the GLP-2 analogues has an effect on the viscosity of the formulation. This opens up the possibility of controlling the viscosity of the formulation by changing and/or controlling the acetate concentration. A low-range viscosity liquid formulation is useful clinically as it provides advantages in drug delivery device development and manufacturing by potentially reducing breakage, dosing failure, dosing imprecision and other malfunctions during drug product manufacture and/or patient use. Furthermore, low viscosity may allow a faster injection and/or the use of narrower bore (i.e., higher gauge) needles that in turn may reduce injection discomfort. This opens up the possibility of providing the formulations of the GLP-2 analogue in the form of a drug delivery device, such as a pre-filled syringe, an adjustable dose auto-injector, a disposable auto-injector, a wearable injector or an infusion pump, thereby providing patients with a ready-to-use formulation in a simpler, safer and more patient-friendly device. Controlling the formulation to higher viscosity could be suitable in other drug delivery devices.

In a second study, the present inventors found that during long term storage at 2-8°C of ZP1848 (glepaglutide), the formation of covalently bound oligomers is concentration dependent. However, contrary to the usual situation in which the law of mass action implies that covalent oligomer formation increases with increasing concentration of a peptide drug, the present inventors found that the concentration dependence for oligomer formation is inversely dependent on increasing concentration of the GLP-2 analogue. Without wishing to be bound by any particular theory, the present inventors believe that the reduction in the formation of covalently linked oligomers as GLP-2 analogue concentration increases is a result of the lysine tail of the GLP-2 analogue promoting the formation of self-associated structural assemblies of the native peptide that hinders the formation of covalently bound oligomers in the formulation. This means that the weakly self-associated species are capable of dissociating to release biologically active monomer after administration into a patient, rather than causing a loss of active species as happens when the covalently bound oligomers are formed.

In a third study, the present inventors found that the GLP-2 analogues used in the formulations of the present invention are not compatible with phosphate buffer commonly used in the prior art to reconstituted powdered or lyophilized GLP-2 compositions. This study found that only some buffers were compatible with formulating these GLP-2 analogues such that they were suitable for long term storage in liquid form.

Accordingly, in a first aspect, the present invention provides a stable liquid pharmaceutical formulation, the formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl;
X5 is Ser or Thr;

X11 is Ala or Ser;
$R^2$ is $NH_2$ or OH; and
$Z^1$ and $Z^2$ are independently absent or a peptide sequence of 1-6 amino acid units of Lys;
or a pharmaceutically acceptable salt or derivative thereof;
wherein the formulation comprises:

(a) the GLP-2 analogue at a concentration of about 2 mg/mL to about 30 mg/mL;

(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;

(c) a non-ionic tonicity modifier selected from the group consisting of mannitol, sucrose, glycerol, sorbitol and trehalose at a concentration of about 90 mM to about 360 mM; and

(d) arginine q.s. to provide a formulation having a pH of about 6.6 to about 7.4.

In some embodiments, the formulation contains 5% or less of the GLP-2 analogue in the form of covalently bonded oligomeric products. Alternatively or additionally, the total acetate concentration arising from the GLP-2 analogue in the formulation is less than or equal to 11% acetate per mg GLP-2 analogue. Alternatively or additionally, formation of covalently linked oligomers of the GLP-2 analogue is inversely dependent on the concentration of the GLP-2 analogue in the formulation.

The components of the formulation and their amounts provide a formulation with at least 90% content of the GLP-2 analogue and with less than 10% of chemical degradation products at storage for at least 18 months at 2-8°C.

In a further aspect, the present invention provides an article of manufacture or a kit comprising a container holding the stable pharmaceutical formulation of the present invention.

In a further aspect, the present invention provides a delivery device containing a liquid formulation comprising a GLP-2 analogue of present invention.

In a further aspect, the present invention provides a formulation of the glucagon-like peptide 2 (GLP-2) analogue of the present invention for use in therapy.

In a further aspect, the present invention provides a formulation of the glucagon-like peptide 2 (GLP-2) analogue of the present invention for use in a method for the treatment and/or prevention of a stomach and bowel-related disorder in a human patient.

In a further aspect, the present invention provides a process for producing a stable liquid pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the process comprising formulating (a) the GLP-2 analogue at a concentration of about 2 mg/mL to about 30 mg/mL, (b) with a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products.

In a further aspect, the present invention provides the use of a formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
for providing an liquid pharmaceutical formulation which is stable for 24 months when stored at 2-8°C, wherein the formulation comprises:

(a) the GLP-2 analogue at a concentration of about 2 mg/mL to about 30 mg/mL;

(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;

(c) a non-ionic tonicity modifier selected from the group consisting of mannitol, sucrose, glycerol, sorbitol and trehalose at a concentration of about 90 mM to about 360 mM; and

(d) arginine q.s. to provide a formulation having a pH of about 6.6 to about 7.4.

In a further aspect, the present invention provides a method for modulating the viscosity of a stable liquid pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the method comprises formulating (a) the GLP-2 analogue at a concentration of about 2 mg/mL to about 30 mg/mL, (b) with a buffer selected from the group consisting of a a histidine buffer, mesylate buffer, acetate buffer, glycine buffer, lysine buffer, TRIS buffer, Bis-Tris buffer or MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4; wherein the total acetate concentration arising from the GLP2 analogue in the formulation is less than or equal to 11% acetate per mg GLP-2 analogue and wherein the formulation has a viscosity greater than 0.8 and lower than or equal to 2.0 mPa/sec measured at 25°C.

In a further aspect, the present invention provides a method for reducing the formation of covalently bonded oligomeric products of a glucagon-like peptide 2 (GLP-2) analogue in a stable liquid pharmaceutical formulation comprising a GLP-2 analogue represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the method comprises formulating (a) the GLP-2 analogue at a concentration of about 2 mg/mL to about 30 mg/mL, (b) with a buffer selected from the group consisting of a histidine buffer, mesylate buffer, acetate buffer, glycine buffer, lysine buffer, TRIS buffer, Bis-Tris buffer or MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products. In some cases, in this aspect of the present invention the formation of covalently linked oligomers of the GLP-2 analogue is inversely dependent on the concentration of the GLP-2 analogue in the formulation.

In a further aspect, the present invention provides use of a formulation for reducing the formation of covalently bonded oligomeric products of a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
in an liquid pharmaceutical formulation which is stable for 24 months when stored at 2-8°C,
wherein the formulation comprises:

(a) the GLP-2 analogue at a concentration of about 2 mg/mL to about 30 mg/mL;

(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;

(c) a non-ionic tonicity modifier selected from the group consisting of mannitol, sucrose, glycerol, sorbitol and trehalose at a concentration of about 90 mM to about 360 mM; and

(d) arginine q.s. to provide a formulation having a pH of about 6.6 to about 7.4;

wherein the formulation contains 5% or less of the GLP-2 analogue in the form of covalently bonded oligomeric products. In some cases, in this aspect of the present invention the formation of covalently linked oligomers of the GLP-2 analogue is inversely dependent on the concentration of the GLP-2 analogue in the formulation.

In a further aspect, the present invention provide use of a formulation for modulating the viscosity of a liquid

pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
in an liquid pharmaceutical formulation which is stable for 24 months when stored at 2-8°C,
wherein the formulation comprises:

(a) the GLP-2 analogue at a concentration of about 2 mg/mL to about 30 mg/mL;

(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;

(c) a non-ionic tonicity modifier selected from the group consisting of mannitol, sucrose, glycerol, sorbitol and trehalose at a concentration of about 90 mM to about 360 mM; and

(d) arginine q.s. to provide a formulation having a pH of about 6.6 to about 7.4;

wherein the total acetate concentration arising from the GLP2 analogue in the formulation is less than or equal to 11% acetate per mg GLP-2 analogue and the formulation has a viscosity between 0.8 and 2.0 mPa/sec measured at 25°C.

In a further aspect, the present invention provides a solid composition comprising an acetate salt of a glucagon-like peptide 2 (GLP-2) analogue having the formula:
(H-HGEGTFSSELATILDALAARDFIAWLIATKITDKKKKKK-NH2), x($CH_3COOH$) where x is 1.0 to 8.0.

In a further aspect, the present invention provides a stable aqueous pharmaceutical formulation, the formulation comprising

(a) the solid composition of the present invention at a concentration of about 2 mg/mL to about 30 mg/mL;

(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;

(c) a non-ionic tonicity modifier selected from the group consisting of mannitol, sucrose, glycerol, sorbitol and trehalose at a concentration of about 90 mM to about 360 mM; and

(d) arginine q.s. to provide a formulation having a pH of about 6.6 to about 7.4;

wherein the formulation contains 5% or less of the GLP-2 analogue in the form of covalently bonded oligomeric products and wherein the formulation has a viscosity between 0.8 and 2.0 mPa/sec measured at 25°C.

In all of the aspects of the invention described herein, the buffer may be selected from the group consisting of a histidine buffer, mesylate buffer and acetate buffer.

In all of the aspects of the invention described herein, the non-ionic tonicity modifier may be selected from the group consisting of mannitol, sucrose, glycerol and sorbitol.

In some embodiments, the formulation contains 5% or less of the GLP-2 analogue in the form of covalently bonded oligomeric products. Alternatively or additionally, the total acetate concentration arising from the GLP-2 analogue in the formulation is less than or equal to 11% acetate per mg GLP-2 analogue. Alternatively or additionally, formation of

covalently linked oligomers of the GLP-2 analogue is inversely dependent on the concentration of the GLP-2 analogue in the formulation.

In a further aspect, the present invention relates to a stable liquid pharmaceutical formulation, the formulation comprising a glucagon-like peptide 2 (GLP-2) analogue, wherein the GLP-2 analogue is represented by the formula:

$R^1$- His-Gly-Glu-Gly-X5-Phe-Ser-Ser-Glu-Leu-X11-Thr-Ile-Leu-Asp-Ala-Leu-Ala-Ala-

Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl;
X5 is Ser or Thr;
X11 is Ala or Ser;
$R^2$ is $NH_2$ or OH; and
$Z^2$ is a peptide sequence of 6 amino acid units of Lys;
or a pharmaceutically acceptable salt or derivative thereof, the formulation comprising the components as set out in any one of the aspects of the invention set out herein.

In this aspect of the present invention, the formulations comprising the glucagon-like peptide 2 (GLP-2) analogue, or salts thereof, may be used for the treatment and/or prevention of stomach and bowel-related disorders such as ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS). Alternatively or additionally, the glucagon-like peptide 2 (GLP-2) analogue may be used for the treatment and/or prevention of stomach and bowel-related disorders such radiation enteritis, infectious or post-infectious enteritis, or small intestinal damage due to toxic or other chemotherapeutic agents. In this case, treatment with the GLP-2 analogue may optionally be combined with one or more anti-cancer therapies, and may therefore comprise administering one or more chemotherapeutic agent(s) to the patient or treating the patient with radiation therapy.

In some embodiments of the present invention, in the above formula, X5 is Thr and/or X11 is Ala. Examples of these glucagon-like peptide 2 (GLP-2) analogues include:

ZP1848 H-HGEGTFSSELATILDALAARDFIAWLIATKITDKKKKKK-$NH_2$ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-$NH_2$(SEQ ID NO: 5); or
ZP2530 H-HGEGTFSSELATILDALAARDFIAWLIATKITD-OH (SEQ ID NO: 6).

In some embodiments of the present invention, in the above formula X5 is Ser and/or X11 is Ser. Examples of these glucagon-like peptide 2 (GLP-2) analogues include:

ZP1846 H-HGEGSFSSELSTILDALAARDFIAWLIATKITDKKKKKK-$NH_2$(SEQ ID NO: 7);
ZP1855 H-HGEGSFSSELSTILDALAARDFIAWLIATKITD-$NH_2$ (SEQ ID NO: 8); or
ZP2242 H-HGEGSFSSELSTILDALAARDFIAWLIATKITDK-OH (SEQ ID NO: 9).

Embodiments of the present invention will now be described by way of example and not limitation with reference to the accompanying figures. However, various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

## Brief Description of the Figures

Figure 1 shows a typical chromatogram showing the separation of the oligomers from the ZP1848 peptide.

Figure 2 shows how the viscosity (squares) and hydrodynamic radius (z-average) (circles) varied as a function of acetate concentration after manufacturing of the formulation. The data shows that above 11 % acetate the viscosity and hydrodynamic radius (z-average) start to increase.

Figure 3 shows the evaluation of stability at 20 mg/mL (normalized to 100% at start) using different buffers at 40° for 0 to 3 weeks.

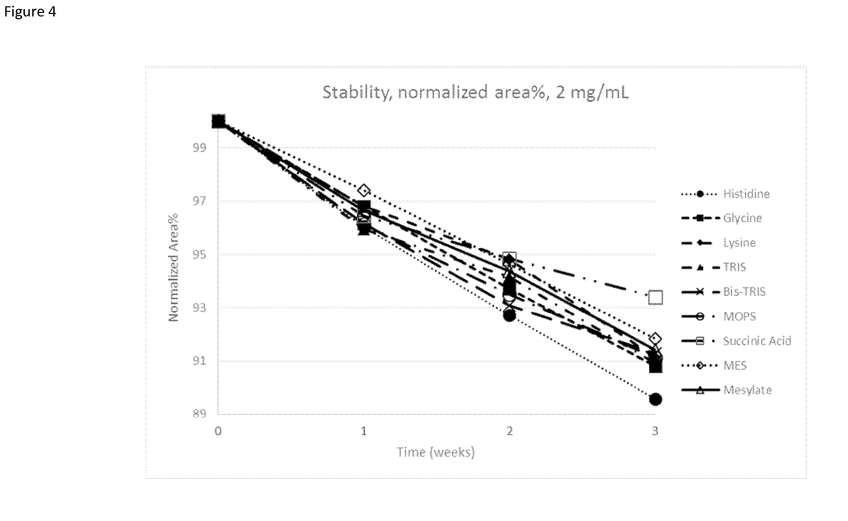

Figure 4 shows the of stability at 2 mg/m (normalized to 100% at start) using different buffers at 40° for 0 to 3 weeks.

Figure 5 shows the evaluation of stability at 20 mg/mL (normalized to 100% at start) using different tonicity agents at 40° for 0 to 3 weeks.

Figure 6 shows the evaluation of stability at 2 mg/mL (normalized to 100% at start) using different tonicity agents at 40° for 0 to 3 weeks.

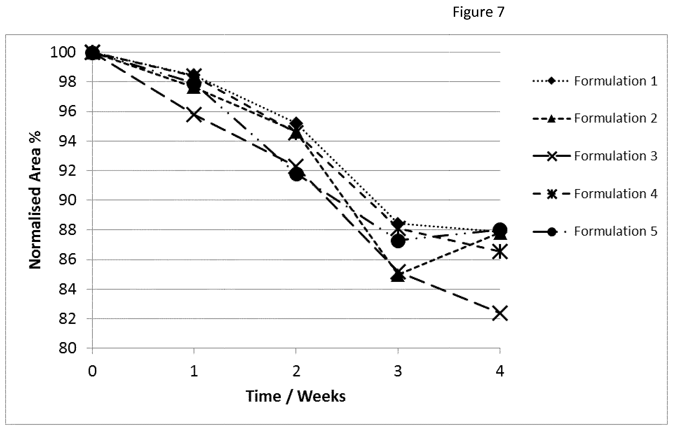

Figure 7 shows the purity of Formulations 1 to 5 using different concentrations of ZP1848 acetate salt, different salt form, different tonicity agent and different buffer.

Figure 8 shows the stability of peptide in combination with different preservatives at 25°C for 13 weeks.

Figure 9 shows the HPLC purity of investigated formulations at 25°C (accelerated conditions).

## Detailed Description of the Invention

### Definitions

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration, However, D-configuration amino acids may also be present.

Preferred compounds of the present invention have at least one GLP-2 biological activity, in particular in causing growth of the intestine. This can be assessed in *in vivo* assays, for example as described in the examples of (e.g.) WO 2006/117565, in which the mass of the intestine, or a portion thereof is determined after a test animal has been treated or exposed to a GLP-2 analogue.

In some aspects of the present invention, the liquid formulations comprising a GLP-2 analogue have a total acetate concentration in the formulation of less than or equal to 11% acetate per mg GLP-2 analogue, and more preferably less than or equal to 10% acetate per mg GLP-2 analogue, more preferably less than or equal to 9% acetate per mg GLP-2 analogue, more preferably less than or equal to 8% acetate per mg GLP-2 analogue, more preferably less than or equal to 7% acetate per mg GLP-2 analogue, more preferably less than or equal to 6% acetate per mg GLP-2 analogue, more preferably less than or equal to 5% acetate per mg GLP-2 analogue, more preferably less than or equal to 4% acetate per mg GLP-2 analogue, more preferably less than or equal to 3% acetate per mg GLP-2 analogue, and more preferably less than or equal to 2% acetate per mg GLP-2 analogue. The acetate concentration in the lyophilized drug substance can be controlled by adjusting the concentration of acetic acid in the mobile phase used during the final chromatographic step. This will result in a drug substance with an acetate content below 11%. Thus, for example, for a formulation having 20 mg/mL of the GLP-2 analogue, the total acetate concentration will be less than or equal to 37 mM. By way of reference 10% total acetate concentration equates to 34 mM, 9% to 30 mM, 8% to 27mM, 7% to 24 mM and 6% to 20nM. The total acetate concentration may be determined using methods known in the art, for example HPLC.

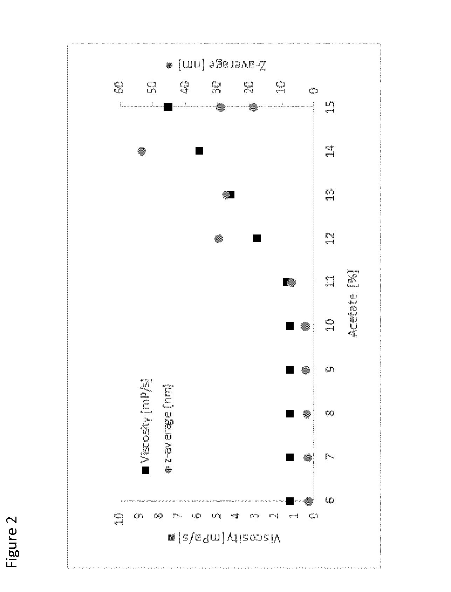

In the examples below, the viscosity of the liquid formulations of the present invention is shown to be dependent on the total acetate concentration. Preferably, the formulations have a viscosity between 0.8 and 2.0 mPa/sec as measured at 25°C. Conveniently, the viscosity may be measured by using *micro*VISC™. In parallel, the hydrodynamic radius may be measured using a Dynamic Light Scattering, DLS, Platereader (Wyatt DynaPro II). Samples were prepared

having a drug substance (DS) of the GLP-2analogue containing 6% acetate and to mimic DS having 7.8-15% acetate, then acetate was added. Data from manufacturing formulations having varied the acetate concentration from 6.7-15% is shown below in Figure 2. The effect of controlling the total acetate concentration is that the injectability of formulations of the present invention can be modulated, for example by reducing the total acetate concentration to provide a less viscous formulation that may be more easily injected.

The liquid formulations according to the present invention are preferably an isosmotic liquid formulation. "Isosmotic" means that the formulations of the present invention have the same or a similar osmotic pressure with bodily fluids. Preferably, the formulations of the present invention have an osmolality of about 300 $\pm$ 60 mOsm as measured by an osmometer.

Additionally or alternatively, the present invention demonstrates that the formation of covalently linked oligomers of the GLP-2 analogue is inversely dependent on the concentration of the GLP-2 analogue in the formulation. As shown in the examples, this amount of covalently bonded oligomers can be determined using size exclusion chromatography and determining the area under the peaks for monomeric GLP-2 analogue and oligomers respectively. This can be done using a Dionex Ultimate3000 HPLC system, giving a linear gradient, at a flow rate of 0.5 mL/min was used for the analysis. The mobile phase consisted of 0.1% TFA in 45% acetonitrile and 55% Milli-Q water. A wavelength of 215 nm was used for detection. This means that the formulations of the present invention generally contain the GLP-2 analogue at a concentration of about 2 mg/mL to about 30 mg/mL, more preferably at a concentration of about 15 mg/mL to about 25 mg/mL, and most preferably at a concentration of about 20 mg/mL. In further embodiments, the present invention generally contain the GLP-2 analogue at a concentration of about 2 mg/mL, 5 mg/mL, 10 mg/mL or 20 mg/mL. In some aspects of the present invention, it is preferred that the concentration of the GLP-2 analogue is selected so that the formulation contains 10% or less, more preferably 5% or less, more preferably 4% or less, more preferably 3% or less, and more preferably 2% or less of the GLP-2 analogue in the form of covalently bonded oligomeric products, preferably after 18 months storage. By way of illustration, the amount of covalently bonded oligomeric product may be in the range of between 2% to 5%, more preferably in the range of between 2% to 4%, and most preferably in the range of between 2% to 3%.

In some cases, the formulation of the present invention may be used in a once or twice daily dosage regime. In some cases, the formulation of the present invention may be used in a once or twice weekly dosage regime. Alternatively or additionally, the dosing regime of the GLP-2 analogues of the present invention may comprise a plurality or course of doses separated in time by 2 days, 2.5 days, 3 days, 3.5 days, 4 days, 5 days, 6 days, 7 days, 8 days, 9 days, 10 days, 11 days or 12 days. In a preferred embodiment, the doses are separated in time by 3 days, 3.5 days, 4 days, 5 days, 6 days, 7 days or 8 days. In a preferred embodiment, doses are separated in time by 3 days, 3.5 days, 4 days or 7 days. As will be appreciated in the art, the time between doses may be varied to some extent so that each and every doses is not separated by precisely the same time. This will often be directed under the discretion of the physician. Thus, doses may be separated in time by a clinically acceptable range of times, e.g. from about 2 days to about 10 days, or from about 3 or 4 days to about 7 or 8 days.

The formulations of the present invention are stable liquid pharmaceutical formulations of GLP-2 analogues. A "stable" formulation is one in which the peptide therein essentially retains its physical stability and/or chemical stability and/or biological activity upon storage. Preferably, the formulation essentially retains its physical and chemical stability, as well as its biological activity upon storage. The storage period is generally selected based on the intended shelf-life of the formulation. The formulations of the present invention are provided as stable liquid formulations, e.g. stable aqueous liquid formulations. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993), for example. In the present invention, "stable" formulations include formulations in which at least 80%, more preferably at least 90%, more preferably at least 95%, more preferably at least 96%, more preferably at least 97%, more preferably at least 98%, and most preferably at least 99% of the GLP-2 analogue is active in the formulation after it has been stored at 2-8°C for at least 18 months.

Stability can be measured at a selected temperature for a selected time period, for example using elevated temperature to reduce the period over which a formulation is tested. Generally, storage at a temperature between 2 to 8°C denotes storage under normal refrigerated conditions. In certain embodiments, the formulation is stable under such conditions for at least 12 months, more preferably at least 18 months, more preferably at least 24 months. Stability can be evaluated qualitatively and/or quantitatively in a variety of different ways, including evaluation of aggregate formation (e.g. using size exclusion chromatography, by measuring turbidity, and/or by visual inspection); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomeriation), clipping/hydrolysis/fragmentation (e.g. hinge region fragmentation), succinimide formation, unpaired cysteine(s), N-terminal extension, C-terminal processing, glycosylation differences, etc.

A peptide "retains its physical stability" in a pharmaceutical formulation if it shows no sign (or very little sign) of

aggregation, precipitation and/or denaturation upon e.g. visual examination of colour and/or clarity, or as measured by UV light scattering, dynamic light scattering, circular dichroism, or by size exclusion chromatography and is considered to still retain its biological activity.

A peptide "retains its chemical stability" in a pharmaceutical formulation, if the chemical stability at a given time is such that the peptide is considered to still retain its biological activity as defined below. Chemical stability can be assessed by detecting and quantifying chemically altered forms of the peptide. Chemical alteration may involve isomerization, oxidation, size modification (e.g. clipping) which can be evaluated using HPLC or size exclusion chromatography, SDS-PAGE and/or mass spectrometry, for example. Other types of chemical alteration include charge alteration n (e.g. occurring as a result of deamidation) which can be evaluated by HPLC or ion-exchange chromatography or icIEF, for example.

GLP-2 analogues

The GLP-2 analogues present in the formulations of the present invention have one or more amino acid substitutions, deletions, inversions, or additions compared with native GLP-2 and as defined above. This definition also includes the synonym terms GLP-2 mimetics and/or GLP-2 agonists. Further, the analogue of the present invention may additionally have chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is $C_1$-$C_4$ alkyl. Furthermore, one or more side groups, or terminal groups, may be protected by protective groups known to the ordinarily-skilled peptide chemist. The α-carbon of an amino acid may be mono- or di-methylated.

In some aspects, the liquid formulations of the present invention employ a glucagon-like peptide 2 (GLP-2) analogue represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl;
X5 is Ser or Thr;
X11 is Ala or Ser;
$R^2$ is $NH_2$ or OH; and
$Z^1$ and $Z^2$ are independently absent or a peptide sequence of 1-6 amino acid units of Lys;
or a pharmaceutically acceptable salt or derivative thereof.

In some embodiments of the present invention, in the above formula, X5 is Thr and/or X11 is Ala. Examples of these glucagon-like peptide 2 (GLP-2) analogues include:

ZP1848 H-HGEGTFSSELATILDALAARDFIAWLIATKITDKKKKKK-$NH_2$ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-$NH_2$(SEQ ID NO: 5); or
ZP2530 H-HGEGTFSSELATILDALAARDFIAWLIATKITD-OH (SEQ ID NO: 6).

In an embodiment of the present invention, the glucagon-like peptide 2 (GLP-2) analogue is ZP1848 H-HGEGTFSSELATILDALAARDFIAWLIATKITDKKKKKK-$NH_2$ (SEQ ID NO: 1).

In some embodiments of the present invention, in the above formula X5 is Ser and/or X11 is Ser. Examples of these glucagon-like peptide 2 (GLP-2) analogues include:

ZP1846 H-HGEGSFSSELSTILDALAARDFIAWLIATKITDKKKKKK-$NH_2$(SEQ ID NO: 7);
ZP1855 H-HGEGSFSSELSTILDALAARDFIAWLIATKITD-$NH_2$ (SEQ ID NO: 8); or

ZP2242 H-HGEGSFSSELSTILDALAARDFIAWLIATKITDK-OH (SEQ ID NO: 9).

In an embodiment of the present invention, the glucagon-like peptide 2 (GLP-2) analogue is ZP1846 H-HGEGSFSSELSTILDALAARDFIAWLIATKITDKKKKKK-$NH_2$(SEQ ID NO: 7).

It should be understood that the peptides (drug substance) of the invention might also be provided in the form of a salt or other derivative. Salts include pharmaceutically acceptable salts, such as acid addition salts and basic salts. Examples of acid addition salts include hydrochloride salts, citrate salts, chloride salts and acetate salts. Preferably, the salt is acetate. In general, it is preferred that the salt is not a chloride salt. Examples of basic salts include salts where the cation is selected from alkali metals, such as sodium and potassium, alkaline earth metals, such as calcium, and ammonium ions $^{+}N(R^3)_3(R^4)$, where $R^3$ and $R^4$ independently designates optionally substituted $C_{1-6}$-alkyl, optionally substituted $C_{2-6}$-alkenyl, optionally substituted aryl, or optionally substituted heteroaryl. Other examples of pharmaceutically acceptable salts are described in "Remington's Pharmaceutical Sciences",17th edition. Ed. Alfonso R. Gennaro (Ed.), Mark Publishing Company, Easton, PA, U.S.A., 1985 and more recent editions, and in the Encyclopaedia of Pharmaceutical Technology.

In preferred embodiments, the acetate salt of a GLP-2 analogue of the invention is selected from the group consisting of ZP1848-acetate, ZP2949-acetate, ZP2711-acetate, ZP2469-acetate, ZP1857-acetate, ZP2530-acetate, ZP1846-acetate, ZP1855-acetate and ZP2242-acetate. In the present context, the term "ZP1848-acetate" refers to the ZP1848 molecule is in the form of an acetate salt. The acetate salts of GLP-2 analogues may be represented by the formula (GLP-2 analogue), x($CH_3COOH$) where x is 1.0 to 8.0, i.e. where x is 1.0, 2.0, 3.0, 4.0, 5.0, 6.0, 7.0 or 8.0. In any composition of the acetate salts of the GLP-2 analogues, there may be molecules with different number of acetate molecules so that x is not necessarily a whole integer. In some cases, x is from 4.0 to 8.0, x is from 6.0 to 8.0, or x is from 4.0 to 6.5. In some cases is from x is from 4.0 to 6.0, x is from 2.0 to 7.0, x is from 3.0 to 6.0, x is from 4.0 to 6.0 or x is 4.0 to 8.0.

In a preferred embodiment, the GLP-2 analogue is ZP1848-acetate or H-HGEGTFSSELATILDALAARDFIAWLIATKITDKKKKKK-$NH_2$ acetate (SEQ ID NO: 1) or (H-HGEGTFSSELATILDALAARDFIAWLIATKITDKKKKKK-$NH_2$), x($CH_3COOH$) where x is 1.0 to 8.0.

Accordingly, in a further aspect, the present invention provides solid compositions comprising an acetate salt of a glucagon-like peptide 2 (GLP-2) analogue. The solid compositions are useful for formulating with the excipients used to make the liquid formulations of the present invention. In one embodiment, the present invention provides a solid composition comprising an acetate salt of a glucagon-like peptide 2 (GLP-2) analogue having the formula: (H-HGEGTFSSELATILDALAARDFIAWLIATKITDKKKKKK-NH2), x($CH_3COOH$) where x is 1.0 to 8.0.

An upper limit of 8.0 acetate molecules per GLP-2 analogue equates to an acetate content of less than 11% acetate and may be formulated to have a viscosity between 0.8 and 2.0 mPa/sec measured at 25°C.

The range of the number of acetate molecules associated with each molecule of the GLP-2 analogues defines a molecular weight range for this component of the formulation. For example, for the acetate salts of ZP1848, the range of the number of acetate molecules associated with each molecule of the GLP-2 analogues defines a molecular weight range of the ZP1848-acetate. By way of example, 1 acetate equivalent with each molecule of ZP1848 provides a molecular weight = 4316 + 60 = 4376 Da. Accordingly, the molecular weights for increasing acetate equivalents with ZP1848 are as follows: 1 acetate equivalent = 4376 Da; 2 acetate equivalents = 4436 Da; 3 acetate equivalents = 4496 Da; 4 acetate equivalents =4556 Da; 5 acetate equivalents = 4616 Da; 6 acetate equivalents = 4676 Da; 7 acetate equivalents = 4736 Da and 8 acetate equivalents = 4796 Da. This in turn defines molecular weight ranges as follows: 1-8 acetate equivalents = 4376 Da - 4796 Da; 4-8 acetate equivalents = 4556 Da - 4796 Da and 6-8 acetate equivalents = 4676 Da - 4796 Da.

Other derivatives of the GLP-2 analogues of the invention include coordination complexes with metal ions such as $Mn^{2+}$ and $Zn^{2+}$, esters such as *in vivo* hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed *in situ* releasing active compound or serve to inhibit clearance of the drug *in vivo.*

$Z^1$ and $Z^2$ are independently present and/or absent or a peptide sequence of 1-6 amino acid units of Lys, i.e. 1, 2, 3, 4, 5 or 6 Lys residues. The Lys residues may have either D- or L-configuration, but have an L-configuration. Particularly preferred sequences Z are sequences of four, five or six consecutive lysine residues, and particularly six consecutive lysine residues. Exemplary sequences Z are shown in WO 01/04156. In certain embodiments, $Z^1$ is absent. In such cases, $Z^2$ may be either present or absent.

Formulations of the GLP-2 analogues

The formulation of the GLP-2 analogues is a ready-to-use formulation. The term "ready-to-use" as used herein

refers to a formulation that does not require constitution or dilution with a prescribed amount of diluent, e.g., water for injection or other suitable diluent, before use by the designated route of administration.

As described herein, the liquid formulations of the GLP-2 analogues of the present invention include a buffer, a non-ionic tonicity modifier and arginine q.s. to provide the pH of the final formulation. In accordance with normal pharmaceutical practice, the formulations of the present invention are sterile and/or free from reducing agent. In some cases, the liquid formulations of the present invention are aqueous, liquid formulations. In some cases, the liquid formulations of the present invention are non-aqueous, liquid formulations.

The term "buffer" as used herein denotes a pharmaceutically acceptable excipient which stabilizes the pH of a pharmaceutical formulation. Suitable buffers are well known in the art and can be found in the literature. The screening experiments in the examples show that the formulations of the present invention preferably include a buffer selected from a histidine buffer, mesylate buffer, acetate buffer, glycine buffer, lysine buffer, TRIS buffer, Bis-Tris buffer and MOPS buffer as these buffers provided stable formulations in which the GLP-2 analogues dissolved and did not become viscous, cloudy or precipitate the peptide drug. In preferred embodiments, the buffer is a histidine buffer, e.g. L-histidine. Generally, the buffer will be present at a concentration of about 5 mM to about 50 mM, more preferably at a concentration of about 5 mM to about 25 mM, and most preferably at a concentration of about 15 mM. Based on the experiments in the present application, preferably the buffer is not a phosphate buffer, a citrate buffer, citrate/Tris buffer and/or succinate buffer.

The term "tonicity modifier" as used herein denotes pharmaceutically acceptable tonicity agents that are used to modulate the tonicity of the formulation. The formulations of the present invention are preferably isosmotic, that is they have an osmotic pressure that is substantially the same as human blood serum. The tonicity modifiers used in the formulations are preferably non-ionic tonicity modifiers and are preferably selected from the group consisting of mannitol, sucrose, glycerol, sorbitol and trehalose. A preferred non-ionic tonicity modified is mannitol, e.g. D-mannitol. The concentration of the tonicity modifier will be dependent on the concentration of other components of the formulation, especially where the formulation is intended to be isosmotic. Typically, the non-ionic tonicity modifier will be employed at a concentration of about 90 mM to about 360 mM, more preferably at a concentration of about 150 mM to about 250 mM, and most preferably at a concentration of about 230 mM.

Generally, the components and amounts of the liquid formulations of the present invention are chosen to provide a formulation with a pH of about 6.6 to about 7.4, more preferably a pH of about 6.8 to about 7.2, and most preferably a pH of about 7.0. Arginine may be added *quantum sufficit (q.s.)* to adjust pH so that it is within a desired pH range. From the experiments shown in the examples, it is preferred that the pH adjustment is not done using hydrochloric acid or sodium hydroxide.

In one embodiment, the liquid formulations of the present invention consists of the GLP-2 analogue at a concentration of about 2 mg/mL to about 30 mg/mL a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM, a non-ionic tonicity modifier selected from the group consisting of mannitol, sucrose, glycerol, sorbitol and trehalose at a concentration of about 90 mM to about 360 mM, arginine q.s. to provide a pH of about 6.6 to about 7.4.

In one embodiment, the liquid formulations of the present invention consists of the GLP-2 analogue at a concentration of about 2 mg/mL to about 30 mg/mL, a buffer selected from the group consisting of a histidine buffer, mesylate buffer and acetate buffer, the buffer being present at a concentration of about 5 mM to about 50 mM, a non-ionic tonicity modifier selected from the group consisting of mannitol, sucrose, glycerol and sorbitol at a concentration of about 90 mM to about 360 mM, arginine q.s. to provide a pH of about 6.6 to about 7.4.

In a further embodiment, the liquid formulations of the present invention comprises the GLP-2 analogue at a concentration of about 20 mg/mL, histidine buffer at a concentration of about 15 mM, mannitol at a concentration of about 230 mM, and arginine q.s. to provide a pH of about 7.0.

In a further embodiment, the liquid formulations of the present invention comprises the GLP-2 analogue at a concentration of about 20 mg/mL, histidine buffer at a concentration of about 15 mM, mannitol at a concentration of about 230 mM and the pH is about 7.0.

In a further embodiment, the liquid formulations of the present invention comprises ZP1848-acetate or H-HGEGTFSSELATILDALAARDFIAWLIATKITDKKKKKK-$NH_2$ acetate (SEQ ID NO: 1) at a concentration of about 20 mg/mL, histidine buffer at a concentration of about 15 mM, mannitol at a concentration of about 230 mM, and arginine q.s. to provide a pH of about 7.0.

In a further embodiment, the liquid formulations of the present invention comprises ZP1848-acetate or H-HGEGTFSSELATILDALAARDFIAWLIATKITDKKKKKK-$NH_2$ acetate (SEQ ID NO: 1) at a concentration of about 20 mg/mL, histidine buffer at a concentration of about 15 mM, mannitol at a concentration of about 230 mM and the pH is about 7.0.

In a further embodiment, the liquid formulations of the present invention comprises an acetate salt of a glucagon-like peptide 2 (GLP-2) analogue having the formula: (H-HGEGTFSSELATILDALAARDFIAWLIATKITDKKKKKK-NH2), x($CH_3COOH$) where x is 1.0 to 8.0., at a concentration of about 20 mg/mL, histidine buffer at a concentration of about 15

mM, mannitol at a concentration of about 230 mM and the pH is about 7.0.

In a further embodiment, the liquid formulations of the present invention comprises an acetate salt of a glucagon-like peptide 2 (GLP-2) analogue having the formula: (H-HGEGTFSSELATILDALAARDFIAWLIATKITDKKKKKK-NH2), x($CH_3COOH$) where x is 1.0 to 8.0., at a concentration of about 20 mg/mL, histidine buffer at a concentration of about 15 mM, mannitol at a concentration of about 230 mM and the pH is about 7.0, in a once or twice daily dosing regimen.

In a further embodiment, the liquid formulations of the present invention comprises an acetate salt of a glucagon-like peptide 2 (GLP-2) analogue having the formula: (H-HGEGTFSSELATILDALAARDFIAWLIATKITDKKKKKK-NH2), x($CH_3COOH$) where x is 1.0 to 8.0., at a concentration of about 20 mg/mL, histidine buffer at a concentration of about 15 mM, mannitol at a concentration of about 230 mM and the pH is about 7.0, in a once or twice weekly dosing regimen.

In a further embodiment, the liquid formulations of the present invention comprises ZP1846 H-HGEGSFSSEL-STILDALAARDFIAWLIATKITDKKKKKK-NH2 (SEQ ID NO: 7); at a concentration of about 20 mg/mL, histidine buffer at a concentration of about 15 mM, mannitol at a concentration of about 230 mM, and arginine q.s. to provide a pH of about 7.0.

In a further embodiment, the liquid formulations of the present invention comprises ZP1846 H-HGEGSFSSEL-STILDALAARDFIAWLIATKITDKKKKKK-$NH_2$(SEQ ID NO: 7); at a concentration of about 20 mg/mL, histidine buffer at a concentration of about 15 mM, mannitol at a concentration of about 230 mM and the pH is about 7.0.

In some cases, the liquid formulations of the present invention further comprise a preservative. In some cases, the preservative is one selected from the group consisting of benzalkonium chloride, chloro butanol, methyl paraben and potassium sorbate. Generally, the preservative is present in a concentration of about 0.1 % to about 1% of the final formulation volume.

In a further embodiment, the liquid formulation is selected from the group consisting of an aqueous liquid formulation, a liquid formulation in various hydrophilic or hydrophobic solvents, an emulsion and a liquid suspension. In a preferred embodiment, the liquid formulation is an aqueous liquid formulation.

By way of example, the liquid formulations of the present invention may be prepared by mixing stock solutions of the GLP-2 analogue, the buffer, the non-ionic tonicity modifier and optionally the preservative in water, optionally diluting the resulting solution and adjusting to the target pH. Conveniently, the solutions of the buffer and the non-ionic tonicity modifier may first be mixed to provide a desired concentration of each excipient. The solution of the GLP-2 analogue may then be added, and if necessary the pH adjusted, for examples using acetic acid/0.5 M L-arginine. Water was added up to the final volume.

Preferably, the glucagon-like peptide 2 (GLP-2) analogue are administered to patients parenterally, preferably by injection, most typically by subcutaneous injection, intramuscular injection, intravenous injection or intraperitoneal injection. Administration by subcutaneous injection is preferred. The injection may be carried out by a physician, nurse or other healthcare professional, or may be self-administered by the patient. As set out herein, in some aspects, the formulations of the present invention have a viscosity that facilitates loading of the formulation into a pre-filled syringe, an injection pen or other injector device. This may have the advantage of pre-determining the dose of the formulation administered to the patient, e.g. without the need for measurement from a multi-use vial. Accordingly, in other aspects, the present invention provides an article of manufacture or a kit comprising a container holding the stable, such as e.g. an aqueous stable pharmaceutical formulation of the GLP-2 analogue according to the present invention or a pre-filled syringe or injector device or injector pen containing an aqueous liquid formulation comprising the GLP-2 analogue according to the present invention.

Medical Conditions

The GLP-2 analogue formulations of the present invention are useful as a pharmaceutical agent for preventing or treating an individual suffering from gastro-intestinal disorders, including the upper gastrointestinal tract of the oesophagus by administering an effective amount of a GLP-2 analogue, or a salt thereof as described herein. The stomach and intestinal-related disorders include ulcers of any aetiology (e.g., peptic ulcers, drug-induced ulcers, ulcers related to infections or other pathogens), digestion disorders, malabsorption syndromes, short-bowel syndrome, cul-de-sac syndrome, inflammatory bowel disease, celiac sprue (for example arising from gluten induced enteropathy or celiac disease), tropical sprue, hypogammaglobulinemic sprue, enteritis, ulcerative colitis, small intestine damage, and chemotherapy induced diarrhoea/mucositis (CID).

As mentioned above, in general individuals who would benefit from increased small intestinal mass and consequent and/or maintenance of normal small intestine mucosal structure and function are candidates for treatment with the present GLP-2 analogues. Particular conditions that may be treated with GLP-2 analogue include the various forms of sprue including celiac sprue which results from a toxic reaction to alpha-gliadin from heat and may be a result of gluten-induced enteropathy or celiac disease, and is marked by a significant loss of villae of the small bowel; tropical sprue which results from infection and is marked by partial flattening of the villae; hypogammaglobulinemic sprue which is observed commonly in patients with common variable immunodeficiency or hypogammaglobulinemia and is marked by significant decrease in villus height. The therapeutic efficacy of the GLP-2 analogue treatment may be monitored by enteric

biopsy to examine the villus morphology, by biochemical assessment of nutrient absorption, by patient weight gain, or by amelioration of the symptoms associated with these conditions.

Another particular condition which may be treated with the GLP-2 analogues of the invention, or for which the GLP-2 analogues may be useful therapeutically and/or prophylactically is short bowl syndrome (SBS), also known as short gut syndrome or simply short gut, which results from surgical resection, congenital defect or disease-associated loss of absorption in the bowel in which patients are subsequently unable to maintain fluid, electrolyte, and nutrient balances on a conventional diet. Despite an adaptation that occurs generally in the two years after resection, SBS patients have reduced dietary uptake and fluid loss.

Other conditions that may be treated with the GLP-2 analogues of the invention, or for which the GLP-2 analogues may be useful prophylactically, include in addition to the above mentioned radiation enteritis, infectious or post-infectious enteritis, and small intestinal damage due to cancer-chemotherapeutic or toxic agents.

The GLP-2 analogues may also be used for the treatment of malnutrition, for example cachexia and anorexia.

A particular embodiment of the invention is concerned with using the present peptides for the prevention and/or treatment of intestinal damage and dysfunction. Such damage and dysfunction is a well-known side effect of cancer-chemotherapy treatment. Chemotherapy administration is frequently associated with unwanted side effects related to the gastronintestinal system such as mucositis, diarrhoea, bacterial translocation, malabsorption, abdominal cramping, gastrointestinal bleeding and vomiting. These side effects are clinical consequences of the structural and functional damage of the intestinal epithelium and frequently make it necessary to decrease the dose and frequency of chemotherapy.

Administration of the present GLP-2 peptide analogues may enhance trophic effect in the intestinal crypts and rapidly provide new cells to replace the damaged intestinal epithelium following chemotherapy. The ultimate goal achieved by administering the present peptides is to reduce the morbidity related to gastrointestinal damage of patients undergoing chemotherapy treatment while creating the most optimal chemotherapy regime for the ftreatment of cancer. Concomitant prophylactic or therapeutic treatment may be provided in accordance with the present invention to patients undergoing or about to undergo radiation therapy.

The stem cells of the small intestinal mucosa are particularly susceptible to the cytotoxic effects of chemotherapy due to their rapid rate of proliferation (Keefe et al., Gut, 47: 632-7, 2000). Chemotherapy-induced damage to the small intestinal mucosa is clinically often referred to as gastrointestinal mucositis and is characterized by absorptive and barrier impairments of the small intestine. For example, it has been shown that, the broadly used chemotherapeutic agents, 5-FU, irinotecan and methothrexate increase apoptosis leading to villus atrophy and crypt hypoplasia in the small intestine of rodents (Keefe et al., Gut 47: 632-7, 2000; Gibson et al., J Gastroenterol. Hepatol. Sep;18(9):1095-1100, 2003; Tamaki et al., J. Int. Med. Res. 31(1):6-16, 2003). Chemotherapeutic agents have been shown to increase apoptosis in intestinal crypts at 24 hours after administration and subsequently to decrease villus area, crypt length, mitotic count per crypt, and enterocyte height three days after chemotherapy in humans (Keefe et al., Gut, 47: 632-7, 2000). Thus, structural changes within the small intestine directly lead to intestinal dysfunction and in some cases diarrhoea.

Gastrointestinal mucositis after cancer chemotherapy is an increasing problem that is essentially untreatable once established, although it gradually remits. Studies conducted with the commonly used cytostatic cancer drugs 5-FU and irinotecan have demonstrated that effective chemotherapy with these drugs predominantly affects structural integrity and function of the small intestine while the colon is less sensitive and mainly responds with increased mucus formation (Gibson et al., J. Gastroenterol. Hepatol. Sep;18(9):1095-1100, 2003; Tamaki et al., J Int. Med. Res. 31(1):6-16, 2003).

The formulations of the present invention comprising GLP-2 analogues may be useful in the prevention and/or treatment of gastrointestinal injury and side effects of chemotherapeutic agents. This potentially important therapeutic application may apply to currently used chemotherapeutic agents such as but not limited to: 5-FU, Altretamine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Mesna, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, Vinorelbine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, and Vinorelbine.

Delivery of the Formulations

In some aspects, the present invention relates to a ready-to-use formulation of GLP-2 analogues, intended for parenteral administration, and suitable for use in e.g. vials, pre-filled syringes, infusion pumps, wearable injectors, disposable auto-injectors or adjustable dose auto-injectors.

## Examples

The following examples are provided to illustrate preferred aspects of the invention and are not intended to limit the scope of the invention. The GLP-2 analogues administered according to the dosage regimes described herein can be made according to the methods such as solid phase peptide synthesis described in WO 2006/117565, the content of which is expressly incorporated by reference in its entirety.

*Example 1. Synthesis of ZP1848-acetate and similar GLP-2 analogues*

ZP1848-acetate peptide was synthesized using an Fmoc Solid Phase Peptide Synthesis (SPPS) approach with standard coupling conditions. After completed synthesis, the peptide sequence was deprotected and cleaved from the solid support, and the crude peptide was purified using preparative reversed-phase HPLC. The peptide was converted to the desired acetate salt form by applying a mobile phase during the final chromatographic step with an appropriate concentration of acetic acid and subsequent lyophilization. The resulting drug substance product had an acetate content below 11% or below 8 equivalents of acetate: batch 1 (6% acetate, 4.6 equivalents of acetate), batch 2 (7% acetate, 5.4 equivalents of acetate) and batch 3 (6% acetate, 4.6 equivalents of acetate). This synthesis and purification protocol may be adapted for making other GLP-2 analogues used in the formulations of the present invention.

*Example 2. Investigating the formation of covalently bound oligomers in pharmaceutical formulations of GLP-2 analogue ZP1848-acetate*

*Materials and Methods*

For detection of covalently linked oligomers, a Dionex Ultimate3000 HPLC system, giving a linear gradient, at a flow rate of 0.5 mL/min was used for the analysis. The mobile phase consisted of 0.1% TFA in 45% acetonitrile and 55% Milli-Q water. A wavelength of 215 nm was used for detection. The injection amount was 4 μg of peptide. The column used for the separation of the covalently formed peptides was a TSKgel SuperSW2000 (TSK BioScience) with a 4 μm particle size and dimensions of 300 * 4.6 mm. The overall runtime was 25 minutes. For chemical stability evaluation of the peptide monomer a C18 column with an acidic mobile phase and an acetonitrile gradient was used.

Stock solutions of mannitol (700 mM), L-histidine (200 mM) and ZP1848 peptide (acetate salt; 60 mg/mL) in water (Milli-Q) were prepared. Mannitol and histidine solutions were mixed in amounts appropriate to give 230 mM mannitol and 15 mM histidine. Peptide stock solution was added to a final concentration of 0.2, 2 and 20 mg/mL, respectively. Water was added up to 90% of final volume. If necessary, pH was adjusted to pH 7 using 1 M acetic acid/0.5 M L-arginine. Water was added up to the final volume.

*Results and Discussion*

It is known in the art that increasing the concentration of peptide or protein drugs in a liquid formulation increases the concentration of dimer, trimers and higher order oligomers as a result of mass action effects leading to a higher probability of covalent reactions (see van Maarschalkerweerd et al., Intrinsically Disord. Proteins. 2015; 3(1): e1071302). Thus, the formation of covalent high molecular weight degradation products (cHMWDP) increases as a function of drug substance concentration and has the effect of reducing the amount of biologically active monomeric peptide available in the formulation. This was therefore investigated in formulations of the GLP-2 analogue, ZP1848-acetate.

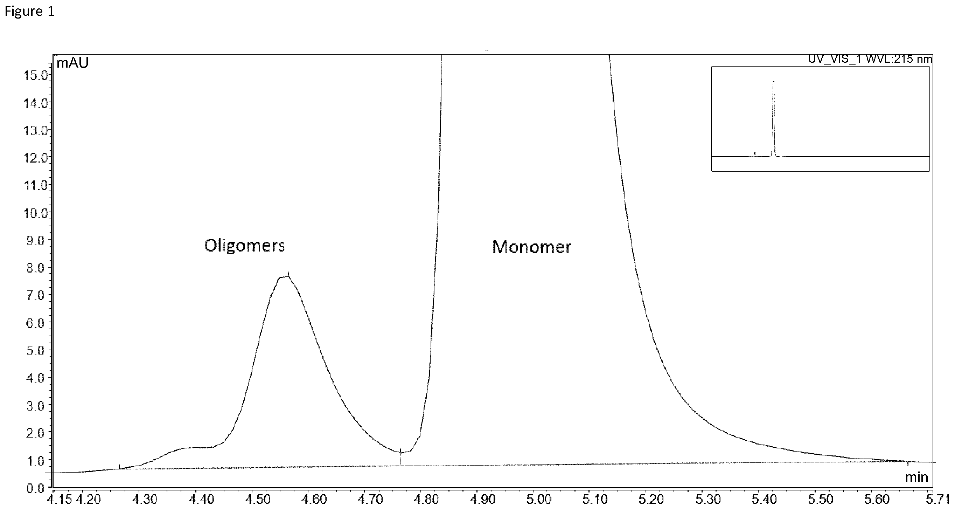

A typical chromatogram on the separation of the oligomers from the ZP1848-acetate monomer is shown in Figure 1. The oligomers of ZP1848-acetate are well separated from the ZP1848-acetate monomer and are all integrated as one peak. The area percentage of the peaks was used to quantify the amount of oligomers, in particular covalently linked dimers and trimers.

Formulations containing 0.2, 2 and 20 mg/mL ZP1848 in the same formulation was analysed after 24 months of storage at 2-8°C. It is primarily formation of dimers (two covalently linked ZP1848-acetate molecules), but also to some extent trimers (verified by LC-MS). The formulation containing 0.2 mg/mL has 2.6% oligomers, 2 mg/mL has 1.91 % and 20.0 mg/mL has 1.35%. The initial value of the amount of oligomer was less than 0.1%.

Table 1: Formation of covalently linked oligomers at long term stability at 2-8°C after 24 months

| Drug product concentration, ZP1848-acetate | Covalently linked oligomers |
|---|---|
| 0.2 mg/mL | 2.60% |
| 2 mg/mL | 1.91% |
| 20 mg/mL | 1.35% |

During long term storage at 2-8°C of ZP1848-acetate (glepaglutide), it has surprisingly been found that the formation of covalently bound oligomers is concentration dependent, but contrary to the general expectation, the concentration dependence for oligomer formation is inversely dependent on increasing concentration of the GLP-2 analogue. Without wishing to be bound by any particular theory, the present inventors believe that the reduction in the formation of covalently linked oligomers as drug concentration increases is a result of the lysine tail of the GLP-2 analogue promoting a competing reaction leading to the formation of higher order species in which the GLP-2 analogue molecules are weakly associated together, rather than being covalently linked. This means that these weakly associated species are capable of dissociating to release biologically active monomer, rather than causing a loss of active species, as happens when the covalently bound oligomers form.

*Example 3: Buffer screening for formulations of GLP-2 analogue ZP1848 acetate*

A study was carried out to examine the effects of different buffer salts on the stability of ZP1848-acetate (4 mg/mL) formulations. The total buffer concentration in the formulations was 20 mM.

*Materials and Methods*

The buffer solutions listed in the Table 2 below were prepared. pH of the buffers were adjusted with 1 M HCl/ 1 M NaOH. ZP1848 peptide (acetate salt) was dissolved in the relevant buffer at 80% of the final sample volume to give 4 mg/mL in the final formulation. If necessary, pH was then adjusted to the desired formulation pH using either 200 mM acetic acid or 100 mM L-arginine. Buffer solution was added up to the final volume. Each formulation was filled in appropriate vials (1 mL/vial) for stability testing.

*Results and Discussion*

Visual appearance showed that all formulations containing citrate buffer, citrate/Tris buffer or succinate buffer were viscous and/or turbid (see Table 2). Acetate buffer (20 mM, pH 5), mesylate buffer (20 mM, pH 6), histidine buffer (15 mM, pH 7) and histidine-arginine (15+5 mM, pH 7) produced formulations which passed visual inspection as being clear and non-viscous.

Table 2: Formulations to screen the effect of different buffers

| Formulation | pH | Buffer | Buffer concentration (mM) | Visual inspection Clear and non-viscous? |
|---|---|---|---|---|
| 1 | 4.0 | Citrate-TRIS | 20 | No |
| 2 | 5.0 | Citrate-TRIS | 20 | No |
| 3 | 6.0 | Citrate-TRIS | 20 | No |
| 4 | 7.0 | Citrate-TRIS | 20 | No |
| 5 | 8.0 | Citrate-TRIS | 20 | No |
| 6 | 5.0 | Succinate | 20 | No |
| 7 | 5.0 | Acetate | 20 | Yes |
| 8 | 5.0 | Histidine | 20 | Yes |
| 9 | 6.0 | Succinate | 20 | No |
| 10 | 6.0 | Mesylate | 20 | Yes |
| 11 | 6.0 | Histidine | 20 | Yes |

(continued)

| Formulation | pH | Buffer | Buffer concentration (mM) | Visual inspection Clear and non-viscous? |
|---|---|---|---|---|
| 12 | 7.0 | Citrate | 20 | No |
| 13 | 7.0 | TRIS | 20 | Yes |
| 14 | 7.0 | Histidine + Arginine | 15 + 5 | Yes |

*Example 4: Phosphate buffer incompatibility with GLP-2 analogue ZP1848-acetate*

*Material and Methods*

Stock solutions of mannitol (700 mM), phosphate buffer (200 mM) and ZP1848-acetate peptide (60.2 mg/mL) in water (Milli-Q) were prepared. Stock solutions were mixed in amounts appropriate to give the formulations shown in the Table 3 below. Water was added up to 90% of final volume. If necessary, pH was then adjusted to the desired formulation pH using 1 M acetic acid/0.5 M L-arginine. Water was added up to the final volume. Sample containers were visually inspected for clarity and viscosity after 24 hours at room temperature.

Table 3: Formulations to test the effect of phosphate buffer

| ZP1848 concentration [mg/mL] | pH | Phosphate [mM] | Mannitol [mM] | Visual inspection Clear and non-viscous? |
|---|---|---|---|---|
| 0 mg/mL | 7.0 | 20 | 230 | Yes |
| 20 mg/mL | 6.5 | 20 | 230 | No |
| 20 mg/mL | 7.0 | 20 | 230 | No |
| 20 mg/mL | 7.0 | 50 | 230 | No |
| 20 mg/mL | 7.5 | 20 | 230 | No |

*Results and Discussion*

Visual inspection showed that formulations of ZP1848-acetate at 20 mg/mL at pH 6.5-7.5 containing 20-50 mM phosphate buffer were turbid and/or highly viscous after 24 hrs at room temperature. Phosphate buffer was therefore concluded to be not compatible with ZP1848-acetate in these formulations.

*Example 5: Effect of acetate content on viscosity of formulations of GLP-2 analogue ZP1848-acetate*

A study was carried out to determine the effect of the acetate content on the viscosity of the ZP1848-acetate formulation.

*Material and Methods*

Samples were prepared using a drug substance (DS) of the GLP-2 analogue ZP1848-acetate containing 6% acetate. Acetate was added to explore the effects of increased acetate content in the range 7.8-15% acetate (see Table 4).

Stock solutions of mannitol (700 mM), acetic acid (1000 mM), histidine (200 mM) and ZP1848-acetate peptide (60 mg/ml) in Milli-Q water were prepared. Stock solutions were mixed in amounts appropriate to give the formulations shown in the Table 4 below. Water was added up to 90% of final volume. If necessary, pH was then adjusted to the desired formulation pH using 250 mM arginine. Water was added up to the final volume. Each formulation was filled in appropriate vials for stability testing.

Vials were visually inspected for clarity and viscosity. The viscosity was measured using a *micro*VISC™ viscosimeter. The hydrodynamic radius was measured using a Wyatt DynaPro II Dynamic Light Scattering (DLS) Platereader. The sample size loaded on the plates was 170 μl.

Table 4: Formulation containing 20 mg/ml of ZP1848-acetate at pH 7 with different acetate concentrations

| Formulation # | Mannitol [mM] | Histidine [mM] | Acetate % | Acetate [mM] |
|---|---|---|---|---|
| 1 | 230 | 15 | 6 | 20.3 |
| 2 | 230 | 15 | 7 | 23.7 |
| 3 | 230 | 15 | 8 | 27.1 |
| 4 | 230 | 15 | 9 | 30.5 |
| 5 | 230 | 15 | 10 | 33.9 |
| 6 | 230 | 15 | 11 | 37.3 |
| 7 | 230 | 15 | 12 | 40.7 |
| 8 | 230 | 15 | 13 | 44.0 |
| 9 | 230 | 15 | 14 | 47.4 |
| 10 | 230 | 15 | 15 | 50.8 |

*Results and Discussion*

The viscosity and hydrodynamic radius of the formulations with varying acetate concentration are shown in Figure 2. The results demonstrate that the viscosity of the ZP1848-acetate formulation unexpectedly increases in a non-linear manner at higher acetate concentration. It is therefore advantageous for controlling the viscosity at a low/unchanged level if the total acetate concentration in the formulation is less than or equal to 11% acetate per mg GLP-2 analogue as this opens up the possibility of providing the formulations of the GLP-2 analogue in the form of a drug delivery device.

*Example 6: Effect of buffer salts on the stability of formulations of GLP-2 analogue ZP1848-acetate at 2 and 20 mg/mL*

A study was carried out to examine the effects of different buffer salts on the stability of ZP1848-acetate (2 and 20 mg/mL) formulations. All buffers were in 15 mM concentration.

*Materials and Methods*

Stock solutions of mannitol (700 mM), L-histidine (200 mM), glycine (400 mM), lysine (200 mM), TRIS (200 mM), bis-TRIS (200 mM), MOPS (100 mM), succinic acid (200 mM), MES (2-(N-morpholino)ethanesulfonic acid) (200 mM), mesylate (200 mM), phosphate (200 mM), and ZP1848 peptide (acetate salt; approx. 50 mg/ml) in water (Milli-Q) were prepared. Excipient solutions were mixed in amounts appropriate to give the formulations shown in Table 5 and Table 6 below. All formulations contained 230 mM mannitol and 15 mM of the buffer agent. Peptide stock solution was added. Water was added up to 90% of final volume. If necessary, pH was adjusted to pH 7 using 1 M acetic acid/0.5 M L-arginine. Water was added up to the final volume. The formulations were filled in vials and placed in a stability study at 40°C.

*Results and Discussion*

According to the observed results, buffer agents histidine, glycine, lysine, TRIS, Bis-TRIS, MOPS, mesylate and MES in 15 mM concentration were acceptable for use in ZP1848-acetate formulations at 2 mg/mL and 20 mg/mL peptide and pH 7.0.

Table 5: Stability of formulations prepared with different buffers

| Formulation | Peptide content | Buffer | Visual inspection Clear and non-viscous? | |
|---|---|---|---|---|
| | | | 0 weeks | 3 weeks (40°C) |
| 1 | 20 | Histidine | Yes | Yes |
| 2 | 20 | Glycine | Yes | Yes |
| 3 | 20 | Lysine | No | Yes |
| 4 | 20 | TRIS | Yes | Yes |

(continued)

| Formulation | Peptide content | Buffer | Visual inspection Clear and non-viscous? | |
|---|---|---|---|---|
| | | | 0 weeks | 3 weeks (40°C) |
| 5 | 20 | Bis-TRIS | Yes | Yes |
| 6 | 2 | Histidine | Yes | Yes |
| 7 | 2 | Glycine | Yes | Yes |
| 8 | 2 | Lysine | Yes | Yes |
| 9 | 2 | TRIS | Yes | Yes |
| 10 | 2 | Bis-TRIS | Yes | Yes |
| 11 | 20 | MOPS | Yes | Yes |
| 12 | 20 | Succinic acid | No | No |
| 13 | 20 | MES | Yes | Yes |
| 14 | 20 | Mesylate | Yes | Yes |
| 15 | 20 | Phosphate | No | No |
| 16 | 2 | MOPS | Yes | Yes |
| 17 | 2 | Succinic acid | Yes | No |
| 18 | 2 | MES | Yes | Yes |
| 19 | 2 | Mesylate | Yes | Yes |
| 20 | 2 | Phosphate | Yes | No |

Table 6: Formation of covalently linked oligomers in formulations using different buffers

| Formulation | Peptide content | Buffer | DLS (Z-average, nm) | |
|---|---|---|---|---|
| | | | 0 weeks | 3 weeks (40°C) |
| 1 | 20 | Histidine | 1.6 | 1.7 |
| 2 | 20 | Glycine | 1.7 | 1.8 |
| 3 | 20 | Lysine | 41.9 | 4.4 |
| 4 | 20 | TRIS | 35.2 | 4.3 |
| 5 | 20 | Bis-TRIS | 1.7 | 1.7 |
| 6 | 2 | Histidine | 2.7 | 3.1 |
| 7 | 2 | Glycine | 2.4 | 3.6 |
| 8 | 2 | Lysine | 3.9 | 4 |
| 9 | 2 | TRIS | 3.8 | 4.1 |
| 10 | 2 | Bis-TRIS | 4.7 | 4.1 |
| 11 | 20 | MOPS | 2.3 | 2.5 |
| 12 | 20 | Succinic acid | 27.4 | 192.5 |
| 13 | 20 | MES | 2.3 | 3.2 |
| 14 | 20 | Mesylate | 25.5 | 4 |
| 15 | 20 | Phosphate | 21.1 | 244.5 |
| 16 | 2 | MOPS | 3.3 | 70.1 |
| 17 | 2 | Succinic acid | 82.5 | 259.3 |
| 18 | 2 | MES | 3.8 | 3.9 |

(continued)

| Formulation | Peptide content | Buffer | DLS (Z-average, nm) | |
|---|---|---|---|---|
| | | | 0 weeks | 3 weeks (40°C) |
| 19 | 2 | Mesylate | 3.7 | 4.1 |
| 20 | 2 | Phosphate | 333.9 | 214.5 |

The formation of covalently linked oligomers were evaluated for the different buffers (Table 6) At 20 mg/mL succinic acid formed a gel after 1 week and could not be evaluated at 2 and 3 weeks of stability. A 2 mg/mL, the same buffer had a significantly higher formation (2.1%) of covalently linked oligomers. The overall trend is that the 2 mg/mL formulations have higher formation of covalently linked oligomers compared to the 20 mg/mL after 3 weeks of accelerated storage at 40°C.

Phosphate buffer and succinate buffer were not compatible with ZP1848-acetate at 2 and 20 mg/mL in these formulations.

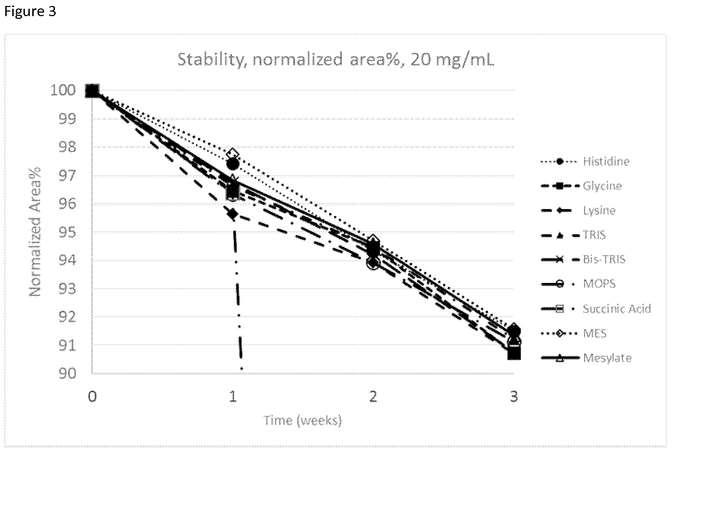

The peptide monomeric stability was evaluated by determining the HPLC purity for 3 weeks of stability at 40°C. The results are presented in Figure 3 and Table 7. Due to gel formation previously described, it was only possible to evaluate succinic acid for the first time-points at 20 mg/mL. For 2 mg/mL results could be obtained for the three tested weeks. Only minor, non significant, differences could be detected between the evaluated buffers. Thus, the choice of buffer does not seem to affect the stability of the peptide monomer.

Table 7: Formation of covalently linked oligomers using different buffers at 40° for 0 to 3 weeks

| Formulation | | Buffer Agent | Time / weeks at 40°C 0 | 1 | 2 | 3 | %/wk |
|---|---|---|---|---|---|---|---|
| 1 | 20 mg/mL | Histidine | 0.14 | 0.40 | 0.55 | 0.69 | 0.18 |
| 2 | | Glycine | 0.15 | 0.39 | 0.53 | 0.64 | 0.16 |
| 3 | | Lysine | 0.15 | 0.38 | 0.51 | 0.64 | 0.16 |
| 4 | | TRIS | 0.10 | 0.38 | 0.51 | 0.63 | 0.17 |
| 5 | | Bis-Tris | 0.15 | 0.42 | 0.58 | 0.72 | 0.19 |
| 11 | | MOPS | 0.14 | 0.38 | 0.49 | 0.59 | 0.15 |
| 12 | | Succinic Acid | 0.14 | 0.40 | Not possible to evaluate | | |
| 13 | | MES | 0.14 | 0.37 | 0.49 | 0.61 | 0.15 |
| 14 | | Mesylate | 0.14 | 0.40 | 0.53 | 0.65 | 0.17 |
| 15 | | Phosphate | Not possible to evaluate | | | | |
| 6 | 2 mg/mL | Histidine | 0.16 | 0.54 | 0.75 | 1.0 | 0.26 |
| 7 | | Glycine | 0.16 | 0.49 | 0.69 | 0.79 | 0.21 |
| 8 | | Lysine | 0.15 | 0.57 | 0.76 | 0.94 | 0.26 |
| 9 | | TRIS | 0.15 | 0.52 | 0.71 | 0.84 | 0.23 |
| 10 | | Bis-Tris | 0.19 | 0.81 | 1.19 | 1.5 | 0.43 |
| 16 | | MOPS | 0.15 | 0.53 | 0.71 | 0.82 | 0.22 |
| 17 | | Succinic Acid | 0.21 | 0.72 | 1.4 | 2.1 | 0.63 |
| 18 | | MES | 0.16 | 0.57 | 0.75 | 0.88 | 0.23 |
| 19 | | Mesylate | 0.16 | 0.60 | 0.82 | 1.0 | 0.26 |
| 20 | | Phosphate | Not possible to evaluate | | | | |

*Example 7: Effect of tonicity modifiers on the stability of formulations of GLP-2 analogue* ZP1848-acetate *at 2 and 20 mg/mL*

A study was carried out to examine the effects of different tonicity modifiers on the stability of ZP1848-acetate (2 and 20 mg/mL) formulations.

*Materials and Methods*

Stock solutions of L-histidine (200 mM), sucrose (730 mM), glycerol (977 mM), D-sorbitol (801 mM), D-(+) Trehalose dehydrate (500 mM), D-mannitol (700 mM) and ZP1848-acetate peptide (acetate salt; approx. 50 mg/ml) in water (Milli-Q) were prepared. Excipient solutions were mixed in amounts appropriate to give the formulations shown in Table 8 below. All formulations contained 15 mM histidine. Peptide stock solution was added as necessary to give the peptide content shown in Table 8. Water was added up to 90% of final volume. If necessary, pH was adjusted to pH 7 using 1 M acetic acid/0.5 M L-arginine. Water was added up to the final volume. Each formulation was filled in vials and placed in stability studies at 40°C. Sample containers were visually inspected for clarity and viscosity and analysed for hydrodynamic radius with DLS (Dynamic Light Scattering) analysis.

*Results and Discussion*

According to the observed results shown in Table 8, mannitol, sucrose, glycerol, sorbitol, and trehalose were

acceptable for use in these formulations with ZP1848-acetate at 2 mg/ml and 20 mg/mL and pH 7.0.

Table 8: Formulations prepared using different tonicity modifiers

| Formulation | Peptide content | Tonicity modifier | Visual inspection Clear and non-viscous? | |
|---|---|---|---|---|
| | | | 0 weeks | 3 weeks (40°C) |
| 1 | 20 | Mannitol | Yes | Yes |
| 2 | 20 | Sucrose | Yes | Yes |
| 3 | 20 | Glycerol | Yes | Yes |
| 4 | 20 | Sorbitol | Yes | Yes |
| 5 | 20 | Trehalose | Yes | Yes |
| 6 | 2 | Mannitol | Yes | Yes |
| 7 | 2 | Sucrose | Yes | Yes |
| 8 | 2 | Glycerol | Yes | Yes |
| 9 | 2 | Sorbitol | Yes | Yes |
| 10 | 2 | Trehalose | Yes | Yes |

Formation of covalent oligomers was measured for formulation 1 to 10 for up to 3 weeks at 40°C. The results are shown in Table 9. Differences in formation of covalent oligomers for the ten formulations can be seen already after 1 week of stability test. Additionally, the rate (slope) is fairly consistent throughout the tested period. Formulation 3 (20 mg/mL - Glycerol), 7 (2 mg/mL - Sucrose), 8 (2 mg/mL -Glycerol), 9 (2 mg/mL - Sorbitol) have a significantly higher formation of covalently linked oligomers than the others. Mannitol gave the lowest formation of covalently linked oligomers. The overall trend is that 2 mg/mL have a higher formation of covalently linked oligomers compared to 20 mg/mL for all the investigated tonicity agents.

Table 9: Formation of covalently linked oligomers of formulation 1-10 at 40°C for 0 to 3 weeks

| | | | | Time / weeks at 40°C | | | |
|---|---|---|---|---|---|---|---|
| Formulation | | | Tonicity agent | 0 | 1 | 2 | 3 | %/wk |
| 1 | | 20 mg/mL | Mannitol | 0.22 | 0.40 | 0.57 | 0.90 | 0.22 |
| 2 | | | Sucrose | 0.27 | 0.50 | 0.84 | 1.3 | 0.34 |
| 3 | | | Glycerol | 0.6 | 2.0 | 2.8 | 4.0 | 1.11 |
| 4 | | | Sorbitol | 0.25 | 0.60 | 0.84 | 1.1 | 0.28 |
| 5 | | | Trehalose | 0.26 | 0.60 | 0.90 | 1.3 | 0.34 |
| 6 | | 2 mg/mL | Mannitol | 0.34 | 0.70 | 0.90 | 1.2 | 0.28 |
| 7 | | | Sucrose | 0.37 | 1.4 | 2.7 | 4.3 | 1.31 |
| 8 | | | Glycerol | 1.8 | 12.2 | 16.4 | 21.5 | 6.34 |
| 9 | | | Sorbitol | 0.51 | 1.9 | 2.8 | 3.8 | 1.08 |
| 10 | | | Trehalose | 0.45 | 1.1 | 1.9 | 2.9 | 0.81 |

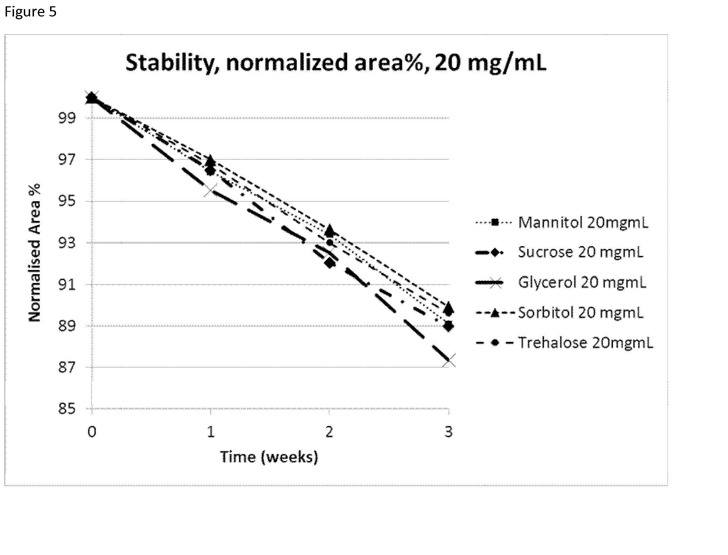

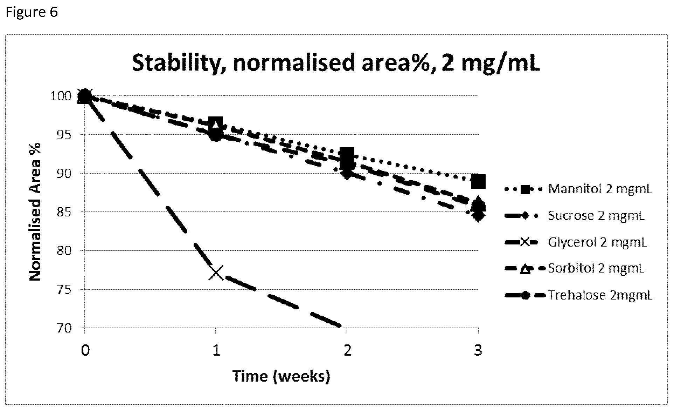

The peptide monomeric stability was evaluated by determining the HPLC purity for 3 weeks of stability at 40°C. Similar to the covalently linked oligomers, the chemical stability when using glycerol is poor and deviates from the other tonicity agents. The results are presented in Figure 5 and Figure 6.

*Example 8: Physical stability impact of acids and bases used for pH adjustment in formulations of GLP-2 analogue ZP1848-acetate*

*Material and Methods*

Stock solutions of mannitol, histidine and ZP1848-acetate peptide in water were prepared. Stock solutions of mannitol and histidine were added to water and mixed, and peptide solution was added to give a final peptide content of 10 mg/mL. Water was added up to 90% of final volume. pH was then adjusted to pH 7 using 250 mM arginine/1 M AcOH or 1 M NaOH/1 M HCl (see Table 10). Water was added up to final volume. Each formulation was filled in vials for stability testing and placed in stability studies at 5°C, 25°C and 40°C. Sample containers were visually inspected for clarity and viscosity.

*Results and Discussion*

The results displayed in Table 10 show that the use of 1 M NaOH/ 1 M HCl for pH adjustment has an adverse effect on the physical stability of the ZP1848-acetate formulation.

Table 10: effect of acid/base on physical stability

| Formula tion | pH adjustment agents | Visual inspection Clear and non-viscous? | | | |
|---|---|---|---|---|---|
| | | Time zero | 40°C 4 weeks | 25°C 26 weeks | 5°C 52 weeks |
| 1 | 0.25 M L-Arg / 1M AcOH | Yes | Yes | Yes | Yes |
| 2 | 1M NaOH / 1M HCl | Yes | Yes | Yes | No (precipitated at 13 weeks) |

*Example 9: Use of ZP1848 peptide acetate salt and ZP1848 peptide chloride salt for formulations of GLP-2 analogue ZP1848 peptide*

A study was carried out to examine the effect of salts using ZP1848 peptide acetate salt and ZP1848 peptide hydrochloride salt in selected ZP1848 formulations. The effects of salt type, concentration, buffer, and tonicity modifiers were examined after accelerated storage at 40°C. Synthesis of ZP1848 peptide sodium salt was attempted, but was not found to be possible.

*Materials and Methods*

Stock solutions of mannitol (700 mM), histidine (200 mM), sorbitol (700 mM), mesylate (200 mM), and ZP1848 peptide solution (chloride salt; approx. 50 mg/mL) in Milli-Q water were prepared. Excipient solutions were mixed in amounts appropriate to give the formulations shown in Table 11 and Table 12 below. Peptide stock solution was added to give the desired final peptide content. Water was added up to 90% of final volume. If necessary, pH was then adjusted to the desired formulation pH using 1 M acetic acid/0.5 M L-arginine. Water was added up to the final volume. Each formulation was filled in vials and placed in stability studies at 40°C. Sample containers were visually inspected for clarity and viscosity and analysed for hydrodynamic radius with DLS.

*Results and Discussion*

The results displayed in Tables 11 and 12 show that the Z-average, viscosity and visual appearance of Formulations 1, 2, 3 and 4 had unchanged stability after 3 weeks at 40°C as evaluated by visual appearance and DLS. Formulation 5 displayed changes in stability over time as evaluated by Z-average, viscosity and visual appearance.

Table 11: Effect of type of peptide salt

| Formulation No: | Peptide salt | Peptide content | Mannitol | Histidine | Sorbitol | Mesylate | Visual inspection Clear and non-viscous? | |
|---|---|---|---|---|---|---|---|---|
| | | | | | | | 0 weeks | 3 weeks (40°C) |
| 1 | ZP1848 acetate salt | 20 | 230 | 15 | - | - | Yes | Yes |
| 2 | ZP1848 chloride salt | 20 | 230 | 15 | - | - | Yes | Yes |
| 3 | ZP1848 chloride salt | 2 | 230 | 15 | - | - | Yes | Yes |
| 4 | ZP1848 chloride salt | 20 | - | 15 | 230 | - | Yes | Yes |
| 5 | ZP1848 chloride salt | 20 | 230 | - | - | 15 | No | No |

Table 12: Effect of type of peptide salt

| Formulation | Peptide salt | Peptide content | Mannitol | Histidine | Sorbitol | Mesylate | DLS (Z-average, nm) | |
|---|---|---|---|---|---|---|---|---|
| | | | | | | | 0 weeks | 3 weeks (40°C) |
| 1 | ZP1848 acetate salt | 20 | 230 | 15 | - | - | 1.5 | 2.1 |
| 2 | ZP1848 chloride salt | 20 | 230 | 15 | - | - | 2.3 | 3.3 |
| 3 | ZP1848 chloride salt | 2 | 230 | 15 | - | - | 2.7 | 2.8 |
| 4 | ZP1848 chloride salt | 20 | - | 15 | 230 | - | 2.2 | 3.3 |
| 5 | ZP1848 chloride salt | 20 | 230 | - | - | 15 | 24.1 | 6.9 |

The chemical stability of Formulations 1 to 5 was followed for up to 4 weeks at 40°C. The obtained purity was at release normalized to 100%. The results are shown in Figure . No significant difference in the peptide monomer chemical stability was seen for Formulations 1, 2, 4 and 5. Formulation 3 showed a slightly lower, but acceptable, chemical stability after 4 weeks and this is most likely caused by the lower concentration of this formulation.

Formation of covalent oligomers was measured for Formulations 1 to 5 for up to 4 weeks at 40°C. The results are shown in Table 13. Differences in formation of covalent oligomers for the five formulations could be seen even after 1 week of testing for stability. Additionally, the rate (slope) for is fairly consistent throughout the tested period. Formulation 1 (20 mg/mL, acetate salt of ZP1848, histidine as tonicity agent) was the most stable formulation with formation of approximately

1.1% of covalent oligomers after 4 week of stability at 40°C. Formulation 5 (20 mg/mL, chloride salt of ZP1848, mesylate as tonicity agent) was the second most stable formulation with approximately 2.1% formation of covalent oligomers after 4 week of stability at 40°C. The third most stable was Formulation 3 (2 mg/mL, chloride salt of ZP1848, histidine as tonicity agent). The fourth most stable is formulation 2 (20 mg/mL, chloride salt of ZP1848, histidine as tonicity agent). The least stable formulation is Formulation 4 (20 mg/mL, chloride salt of ZP1848, sorbitol as tonicity agent).

A slight tendency of lower stability when using sorbitol compared to mannitol has previously been seen for the acetate salt of ZP1848 after 3 weeks at 40°C (0.9% for mannitol and 1.1% for sorbitol) see Example 7. The difference between a sorbitol and mannitol containing formulation is more pronounced when comparing the acetate and chloride salts where the chloride salt and sorbitol containing formulation at 20 mg/mL has approximately 3.9% formation of covalent oligomers. When comparing 2 mg/mL and 20 mg/mL formulation of the chloride salt, the formation of covalent oligomers was 2.4% for 2 mg/mL (0.53% increase per week) and 3.3% for 20 mg/mL (0.75% increase per week) after 4 weeks at 40°C. This is in surprisingly since this is not what is observed for the acetate salt. For the acetate salt at 20 mg/mL the formation of covalent oligomers after 3 weeks at 40°C was 0.9% (0.21% increase per week) whereas the 2 mg/mL is 1.2% (0.27% increase per week). This higher formation of covalently linked oligomers at lower concentrations of the acetate salt is also in agreement with what is seen during long term stability. However, for the chloride salt, the situation was reversed with higher formation of covalently linked oligomers with increase of ZP1848 concentration.

Table 13: Formation of covalently linked oligomers of formulation 1-5 at 40°C for 0 to 4 weeks

| | Time / Weeks at 40°C | | | | | Rate |
|---|---|---|---|---|---|---|
| | **0** | **1** | **2** | **3** | **4** | **%/wk** |
| Formulation 1 | 0.13 | 0.43 | 0.67 | 0.89 | 1.1 | 0.24 |
| Formulation 2 | 0.22 | 1.5 | 2.2 | 2.8 | 3.3 | 0.75 |
| Formulation 3 | 0.22 | 0.99 | 1.5 | 1.9 | 2.4 | 0.53 |
| Formulation 4 | 0.23 | 1.7 | 2.5 | 3.4 | 3.9 | 0.91 |
| Formulation 5 | 0.22 | 0.86 | 1.3 | 1.6 | 2.1 | 0.44 |

*Example 10: Use of ZP1848 peptide acetate salt and preservatives for formulations of GLP-2 analogue ZP1848 peptide at 20 mg/mL*

A study was carried out to examine the compatibility of ZP1848 peptide acetate salt and commonly used preservatives. The effects of preservative and temperature were examined after accelerated storage.

*Materials and Methods*

Stock solutions of mannitol (700 mM), histidine (200 mM) and ZP1848 peptide solution (acetate salt; approx. 50 mg/mL) in Milli-Q water were prepared. Final concentration of peptide was 20 mg/mL, mannitol 230 mM, histidine 15 mM. Preservative solutions were mixed in amounts appropriate to give the formulations shown in Table 14 below. Peptide stock solution was added to give the desired final peptide content. Water was added up to 90% of final volume. If necessary, pH was then adjusted to the desired formulation pH using 1 M acetic acid/0.5 M L-arginine. Water was added up to the final volume. Each formulation was filled in vials. Sample containers were visually inspected for clarity and viscosity and analysed for covalently linked oligomers by SEC, and peptide monomer stability by HPLC.

*Results and Discussion*

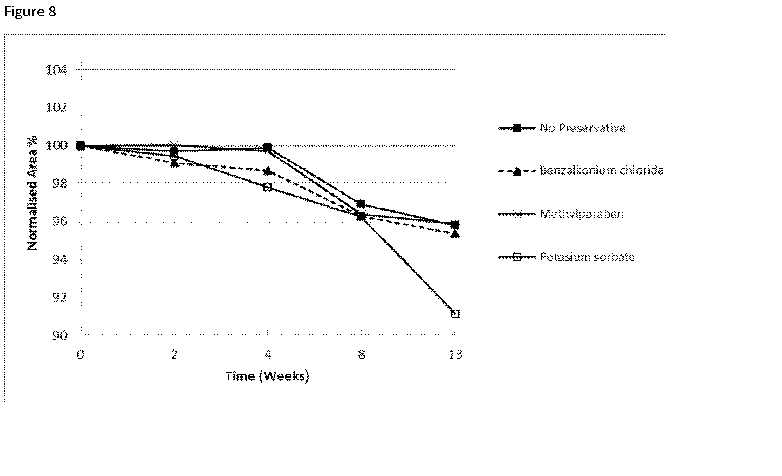

The results from the study are listed in Table 14, Table 15 and Figure 8. The formulations do not seem to be affected by the addition of a preservative compared to the Formulation 1 where no preservative is added.

Chemical stability was evaluated by determination of the covalently linked oligomers and the peptide monomer stability (purity). Formulation 4 (potassium sorbate) has got a higher formation of the covalently linked oligomers but is within the acceptable range. All others formulations have similar amount of covalently linked oligomers. The normalized purity after 13 weeks at 25°C shows that the ZP1848-acetate have similar stability again with formulation 4 having at slightly lower, but acceptable, purity.

Table 14: Effect of preservative screen at 25°C for 26 weeks

| Formulation No: | Benzalkonium chloride | Methyl paraben | Potasium sorbate | Visual inspection Clear and non-viscous? | |
|---|---|---|---|---|---|
| | | | | 0 weeks | 26 weeks (25°C) |
| 1 | - | - | - | Clear | Clear |
| 2 | 0.02% | - | - | Clear | Clear |
| 3 | - | 0.2% | - | Clear | Clear |
| 4 | - | - | 0.2% | Clear | Clear |

Table 15 Formation of covalently linked oligomers for different preservatives at 25°C for 13 weeks

| | Time / Weeks at 25°C | | | | | Rate |
|---|---|---|---|---|---|---|
| | 0 | 2 | 4 | 8 | 13 | %/wk |
| Formulation 1 | 0.12 | 0.27 | 0.35 | 0.47 | 0.62 | 0.04 |
| Formulation 2 | 0.13 | 0.26 | 0.35 | 0.48 | 0.59 | 0.03 |
| Formulation 3 | 0.14 | 0.34 | 0.49 | 0.79 | 1.15 | 0.08 |
| Formulation 4 | 0.14 | 0.93 | 1.73 | 3.46 | 5.4 | 0.41 |

*Example 11: Use of ZP1848 peptide acetate salt and preservatives for formulations of GLP-2 analogue ZP1848 peptide at 2 and 20 mg/mL*

*Materials and methods*

Stock solutions of mannitol (700 mM), histidine (200 mM), and ZP1848 peptide solution (acetate salt; approx. 50 mg/mL) in Milli-Q water were prepared. Final concentration of peptide was 20 and 2 mg/mL, mannitol 230 mM, and histidine 15 mM. Preservative solutions (m-cresol and phenol) were mixed in amounts appropriate to give the formulations shown in Table 14 below. Water was added up to 90% of final volume. If necessary, pH was then adjusted to the desired formulation pH (7.0) using 1 M acetic acid/0.5 M L-arginine. Water was added up to the final volume. Each formulation was filled in vials. Sample containers were visually inspected for clarity and viscosity and analysed for peptide monomer stability by HPLC.

*Results and Discussion*

The results from the study are listed in Table 16 below. All formulations were tested for long term stability at 5°C for 52 weeks. All tested solutions remained clear and non-viscous through the investigated timeframe.

Table 16: Formulations tested for physical appearance after 52 weeks of stability at 5°C

| Formulation No: | mg/mL | m-cresol (mg/mL) | Phenol (mg/mL) | Visual inspection Clear and non-viscous? | |
|---|---|---|---|---|---|
| | | | | 0 weeks | 52 weeks (5°C) |
| 1 | 2 | 0 | 0 | Clear | Clear |
| 2 | 2 | 3.2 | 0 | Clear | Clear |
| 3 | 2 | 0 | 5.5 | Clear | Clear |

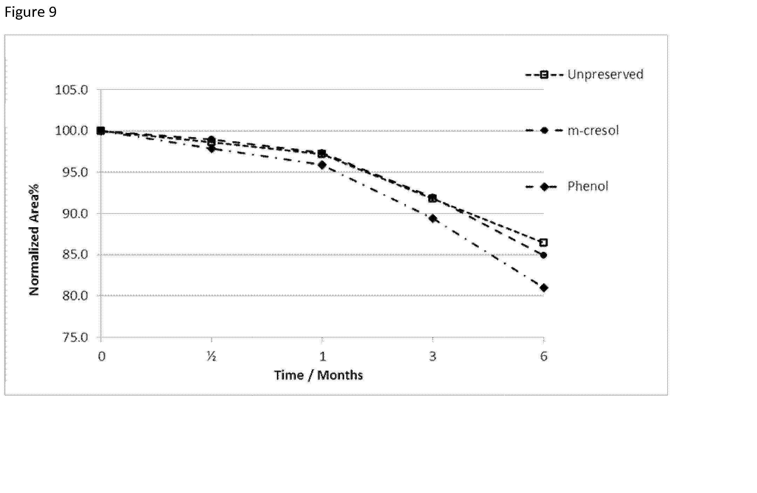

Evaluation of ZP1848-acetate after accelerated stability at 25°C by HPLC are shown in Figure 9. A slightly lower chemical stability is seen for the phenol containing formulation and the m-cresol got a similar chemical stability as the unpreserved formulation. At long term stability at 5°C, no apparent differences can be seen for the samples after 12 months of stability and all samples have a normalized ZP1848-acetate purity above 94% (data not shown). Hence, all investigated formulations are stable for at least 52 weeks of long term stability.

While the present invention has been described in conjunction with the embodiments described above, many equivalent modifications and variations will be apparent to those skilled in the art when given this disclosure. Accordingly, the embodiments of the invention set forth are considered to be illustrative and not limiting. Various changes to the described embodiments may be made without departing from the spirit and scope of the invention. All documents cited herein are expressly incorporated by reference in their entirety for all purposes.

## Claims

**1.** A stable liquid pharmaceutical formulation, the formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl;
X5 is Ser or Thr;
X11 is Ala or Ser;
$R^2$ is $NH_2$ or OH; and
$Z^1$ and $Z^2$ are independently absent or a peptide sequence of 1-6 amino acid units of Lys;
or a pharmaceutically acceptable salt or derivative thereof;
wherein the formulation comprises:

(a) the GLP-2 analogue at a concentration of about 2 mg/mL to about 30 mg/mL;
(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(c) a non-ionic tonicity modifier selected from the group consisting of mannitol, sucrose, glycerol, sorbitol and trehalose at a concentration of about 90 mM to about 360 mM; and
(d) arginine q.s. to provide a formulation having a pH of about 6.6 to about 7.4;

wherein the formulation contains 5% or less of the GLP-2 analogue in the form of covalently bonded oligomeric products.

**2.** The formulation according to clause 1, wherein the total acetate concentration arising from the GLP2 analogue in the formulation is less than or equal to 11% acetate per mg GLP-2 analogue.

**3.** The formulation according to clause 1 or clause 2, wherein formation of covalently linked oligomers of the GLP-2 analogue is inversely dependent on the concentration of the GLP-2 analogue in the formulation.

**4.** The formulation according to any one of clauses 1 to 3, wherein the formulation is an aqueous formulation.

**5.** The formulation according to any one of clauses 1 to 4, wherein the formulation is stable for at least 18 months when stored at 2-8°C.

**6.** The formulation according to any one of the preceding clauses , wherein the formulation has a viscosity between 0.8 and 2.0 mPa/sec measured at 25°C.

**7.** The formulation according to any one of the preceding clauses, wherein the GLP-2 analogue is present in the formulation at a concentration of about 15 mg/mL to about 25 mg/ml.

**8.** The formulation according to any one of the preceding clauses, wherein the formulation is a ready-to-use formulation.

**9.** The formulation according to any one of the preceding clauses , wherein the GLP-2 analogue is present in the

formulation at a concentration of about 20 mg/mL.

**10.** The formulation according to any one of the preceding clauses, wherein the buffer is present in the formulation at a concentration of about 5 mM to about 25 mM.

**11.** The formulation according to any one of the preceding clauses, wherein the buffer is a histidine buffer.

**12.** The formulation according to clause 11, wherein the histidine buffer is present in the formulation at a concentration of about 15 mM.

**13.** The formulation according to any one of the preceding clauses, wherein the non-ionic tonicity modifier is present in the formulation at a concentration of about 150 mM to about 250 mM.

**14.** The formulation according to any one of the preceding clauses, wherein the non-ionic tonicity modifier is mannitol.

**15.** The formulation according to clause 14, wherein the mannitol is present in the formulation at a concentration of about 230 mM.

**16.** The formulation according to any one of the preceding clauses, wherein the formulation has a pH of about 6.8 to about 7.2.

**17.** The formulation according to any one of the preceding clauses , wherein the formulation has a pH of about 7.0.

**18.** The formulation according to any one of the preceding clauses , wherein the formulation consists of the GLP-2 analogue at a concentration of about 2 mg/mL to about 30 mg/ml, a buffer selected from the group consisting of a histidine buffer, mesylate buffer and acetate buffer, the buffer being present at a concentration of about 5 mM to about 50 mM, a non-ionic tonicity modifier selected from the group consisting of mannitol, sucrose, glycerol and sorbitol at a concentration of about 90 mM to about 360 mM, arginine q.s. to provide a pH of about 6.6 to about 7.4.

**19.** The formulation according to any one of the preceding clauses , wherein the formulation comprises the GLP-2 analogue at a concentration of about 20 mg/mL, histidine buffer at a concentration of about 15 mM, mannitol at a concentration of about 230 mM, and arginine q.s. to provide a pH of about 7.0.

**20.** The formulation according to any one of the preceding clauses, wherein the histidine buffer is L-histidine.

**21.** The formulation according to any one of the preceding clauses, wherein the mannitol is D-mannitol.

**22.** The formulation according to any one of the preceding clauses, wherein the arginine acid is L-arginine/acetic acid.

**23.** The formulation according to any one of the preceding clauses , wherein the formulation is free of reducing agent.

**24.** The formulation according to any one of the preceding clauses, wherein the formulation is stable at 2-8°C for at least 6 months, at least 12 months, at least 18 months or at least 24 months.

**25.** The formulation according to clause 24, wherein the GLP-2 analogue in the formulation retains at least about 90% of its biological activity after 18 months of storage 2-8°C.

**26.** The formulation according to any one of the preceding clauses which is sterile.

**27.** The formulation according to any one of the preceding clauses , wherein the formulation is administration to a subject by injection.

**28.** The formulation according to clause 27, wherein the injection is a subcutaneous injection.

**29.** The formulation according to any one of the preceding clauses, wherein the GLP-2 analogue is provided as an acetate salt.

**30.** The formulation according to any one of the preceding clauses, wherein the GLP-2 analogue is ZP1848 or

ZP1848-acetate.

**31.** The formulation according to any one of the preceding clauses, wherein the formulation consist of ZP1848-acetate at a concentration of about 20 mg/mL, histidine buffer at a concentration of about 15 mM, mannitol at a concentration of about 230 mM, and arginine q.s. to provide a pH of about 7.0.

**32.** An article of manufacture or a kit comprising a container holding the stable pharmaceutical formulation of any one of clauses 1 to 31.

**33.** A delivery device containing a liquid formulation comprising a GLP-2 analogue of any one of clauses 1 to 31.

**34.** The delivery device of clause 33, wherein the delivery device is pre-filled syringe, an injector device, an injector pen, an adjustable dose auto-injector, a disposable auto-injector, a wearable injector, an infusion pump.

**35.** A formulation of the glucagon-like peptide 2 (GLP-2) analogue of any one of clauses 1 to 31 for use in therapy.

**36.** A formulation of the glucagon-like peptide 2 (GLP-2) analogue of any one of clauses 1 to 31 for use in a method for the treatment and/or prevention of a stomach and bowel-related disorder in a human patient.

**37.** The formulation of the glucagon-like peptide 2 (GLP-2) analogue for use in the method of treatment and/or prevention of clause 36, wherein the stomach and bowel-related disorder is ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS).

**38.** The formulation of the glucagon-like peptide 2 (GLP-2) analogue for use in the method of treatment and/or prevention of clause 37, wherein the stomach and bowel-related disorder is short bowel syndrome.

**39.** The formulation of the glucagon-like peptide 2 (GLP-2) analogue for use in a method for the treatment according to clause 36, wherein the stomach and bowel-related disorder is radiation enteritis, infectious or post-infectious enteritis, or small intestinal damage due to toxic or other chemotherapeutic agents.

**40.** The formulation of the glucagon-like peptide 2 (GLP-2) analogue for use in a method for the treatment according to clause 39, wherein treatment with the GLP-2 analogue is combined with one or more anti-cancer therapies.

**41.** The formulation of the glucagon-like peptide 2 (GLP-2) analogue for use in a method for the treatment according to clause 40, wherein treatment the anti-cancer therapy comprises administering one or more chemotherapeutic agent(s) to the patient or treating the patient with radiation therapy.

**42.** The formulation of the glucagon-like peptide 2 (GLP-2) analogue for use in a method for the treatment according to clause 40 or clause 41, wherein the formulation is used in the treatment and/or prevention of a side effect of chemotherapy or radiation treatment.

**43.** The formulation of the glucagon-like peptide 2 (GLP-2) analogue for use in a method for the treatment according to clause 42, wherein the side effect of chemotherapy is diarrhoea, abdominal cramping, vomiting or structural and functional damage of the intestinal epithelium resulting from chemotherapy treatment.

**44.** The formulation of the glucagon-like peptide 2 (GLP-2) analogue for use in a method for the treatment according to any one of clauses 40 to 43, wherein the human patient is a patient having SBS-intestinal failure.

**45.** The formulation of the glucagon-like peptide 2 (GLP-2) analogue for use in a method for the treatment according to any one of clauses 40 to 44, wherein the human patient is a patient being on the border between being a patient having SBS-intestinal insufficiency and SBS-intestinal failure.

**46.** The formulation of the glucagon-like peptide 2 (GLP-2) analogue for use in a method for the treatment according to any one of clauses 35 to 45, wherein the method comprises administering the GLP-2 analogue to the patient once weekly.

**47.** The formulation of the glucagon-like peptide 2 (GLP-2) analogue for use in a method for the treatment according to any one of clauses 29 to 43, wherein the method comprises administering the GLP-2 analogue to the patient twice weekly.

**48.** A process for producing a stable liquid pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala- Trp-Leu-Ile-Ala- Thr-Lys-Ile- Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the process comprising formulating (a) the GLP-2 analogue at a concentration of about 2 mg/mL to about 30 mg/mL, (b) with a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products.

**49.** The process according to clause 48, wherein formation of covalently linked oligomers of the GLP-2 analogue is inversely dependent on the concentration of the GLP-2 analogue in the formulation.

**50.** The process according to clause 48 or clause 49, wherein the formulation is stable for at least 18 months when stored at 2-8°C.

**51.** The process according to any one of clauses 48 to 50, wherein the total acetate concentration arising from the GLP2 analogue in the formulation is less than or equal to 11% acetate per mg GLP-2 analogue.

**52.** The process according to any one of clauses 48 to 51, wherein the formulation has a viscosity greater than 0.8 and lower than or equal to 2.0 mPa/sec measured at 25°C.

**53.** Use of a formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala- Trp-Leu-Ile-Ala- Thr-Lys-Ile- Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
for providing an liquid pharmaceutical formulation which is stable for 24 months when stored at 2-8°C, wherein the formulation comprises:

(a) the GLP-2 analogue at a concentration of about 2 mg/mL to about 30 mg/mL;
(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(c) a non-ionic tonicity modifier selected from the group consisting of mannitol, sucrose, glycerol, sorbitol and trehalose at a concentration of about 90 mM to about 360 mM; and
(d) arginine q.s. to provide a formulation having a pH of about 6.6 to about 7.4;

wherein the formulation contains 5% or less of the GLP-2 analogue in the form of covalently bonded oligomeric products.

**54.** The use according to clause 53, wherein formation of covalently linked oligomers of the GLP-2 analogue is inversely dependent on the concentration of the GLP-2 analogue in the formulation.

**55.** The use according to clause 53 or clause 54, wherein the total acetate concentration arising from the GLP2 analogue in the formulation is less than or equal to 11% acetate per mg GLP-2 analogue.

**56.** The use according to any one of clauses 53 to 54, wherein the formulation has a viscosity between 0.8 and 2.0 mPa/sec measured at 25°C.

**57.** A method for modulating the viscosity of a stable liquid pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the method comprises formulating (a) the GLP-2 analogue at a concentration of about 2 mg/mL to about 30 mg/mL, (b) with a buffer selected from the group consisting of a a histidine buffer, mesylate buffer, acetate buffer, glycine buffer, lysine buffer, TRIS buffer, Bis-Tris buffer or MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the total acetate concentration arising from the GLP2 analogue in the formulation is less than or equal to 11% acetate per mg GLP-2 analogue and wherein the formulation has a viscosity greater than 0.8 and lower than or equal to 2.0 mPa/sec measured at 25°C.

**58.** A method for reducing the formation of covalently bonded oligomeric products of a glucagon-like peptide 2 (GLP-2) analogue in a stable liquid pharmaceutical formulation comprising a GLP-2 analogue represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala- Trp-Leu-Ile-Ala- Thr-Lys-Ile- Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser

$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the method comprises formulating (a) the GLP-2 analogue at a concentration of about 2 mg/mL to about 30 mg/mL, (b) with a buffer selected from the group consisting of a histidine buffer, mesylate buffer, acetate buffer, glycine buffer, lysine buffer, TRIS buffer, Bis-Tris buffer or MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products

**59.** The method of clause 58, wherein formation of covalently linked oligomers of the GLP-2 analogue is inversely dependent on the concentration of the GLP-2 analogue in the formulation.

**60.** Use of a formulation for reducing the formation of covalently bonded oligomeric products of a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
in an liquid pharmaceutical formulation which is stable for 24 months when stored at 2-8°C,
wherein the formulation comprises:

(a) the GLP-2 analogue at a concentration of about 2 mg/mL to about 30 mg/mL;
(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(c) a non-ionic tonicity modifier selected from the group consisting of mannitol, sucrose, glycerol, sorbitol and trehalose at a concentration of about 90 mM to about 360 mM; and
(d) arginine q.s. to provide a formulation having a pH of about 6.6 to about 7.4;

wherein the formulation contains 5% or less of the GLP-2 analogue in the form of covalently bonded oligomeric products.

**61.** The use according to clause 60, wherein formation of covalently linked oligomers of the GLP-2 analogue is inversely dependent on the concentration of the GLP-2 analogue in the formulation.

**62.** Use of a formulation for modulating the viscosity of a liquid pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-Ala-

Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl

X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
in an liquid pharmaceutical formulation which is stable for 24 months when stored at 2-8°C,
wherein the formulation comprises:

(a) the GLP-2 analogue at a concentration of about 2 mg/mL to about 30 mg/mL;
(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(c) a non-ionic tonicity modifier selected from the group consisting of mannitol, sucrose, glycerol, sorbitol and trehalose at a concentration of about 90 mM to about 360 mM; and
(d) arginine q.s. to provide a formulation having a pH of about 6.6 to about 7.4;

wherein the total acetate concentration arising from the GLP2 analogue in the formulation is less than or equal to 11% acetate per mg GLP-2 analogue and the formulation has a viscosity between 0.8 and 2.0 mPa/sec measured at 25°C.

**63.** A solid composition comprising an acetate salt of a glucagon-like peptide 2 (GLP-2) analogue having the formula: (H-HGEGTFSSELATILDALAARDFIAWLIATKITDKKKKKK-NH2), x($CH_3COOH$) where x is 1.0 to 8.0.

**64.** The solid composition of clause 63, wherein x is from 4.0 to 6.0.

**65.** The solid composition of clause 63, wherein x is from 2.0 to 7.0.

**66.** The solid composition of clause 63, wherein x is from 3.0 to 6.0.

**67.** The solid composition of clause 63, wherein x is from 4.0 to 6.0.

**68.** The solid composition of clause 63, wherein x is from 4.0 to 8.0.

**69.** A stable aqueous pharmaceutical formulation, the formulation comprising

(a) the solid composition of clauses 63 to 67 at a concentration of about 2 mg/mL to about 30 mg/mL;
(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(c) a non-ionic tonicity modifier selected from the group consisting of mannitol, sucrose, glycerol, sorbitol and trehalose at a concentration of about 90 mM to about 360 mM; and
(d) arginine q.s. to provide a formulation having a pH of about 6.6 to about 7.4;

wherein the formulation contains 5% or less of the GLP-2 analogue in the form of covalently bonded oligomeric products and wherein the formulation has a viscosity between 0.8 and 2.0 mPa/sec measured at 25°C.

**70.** The formulation of clause 69, wherein the formulation is stable for at least 18 months when stored at 2-8°C.

**71.** The formulation of clause 69 or clause 70, wherein the GLP-2 analogue in the formulation retains at least about 90% of its biological activity after 18 months of storage 2-8°C.

**1.** A stable liquid pharmaceutical formulation, the formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-

Ala-Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl;
X5 is Ser or Thr;
X11 is Ala or Ser;
$R^2$ is $NH_2$ or OH; and
$Z^1$ and $Z^2$ are independently absent or a peptide sequence of 1-6 amino acid units of Lys;
or a pharmaceutically acceptable salt or derivative thereof;
wherein the formulation comprises:

(a) the GLP-2 analogue at a concentration of about 2 mg/mL to about 30 mg/mL;
(b) a buffer selected from the group consisting of a histidine buffer, mesylate buffer, acetate buffer, glycine buffer, lysine buffer, TRIS buffer, Bis-Tris buffer and MOPS buffer, the buffer being present at a concentration of about 5 mM to about 50 mM;
(c) a non-ionic tonicity modifier selected from the group consisting of mannitol, sucrose, glycerol, sorbitol and trehalose at a concentration of about 90 mM to about 360 mM; and
(d) arginine q.s. to provide a formulation having a pH of about 6.6 to about 7.4.

**2.** The formulation according to claim 1:

wherein the formulation contains 5% or less of the GLP-2 analogue in the form of covalently bonded oligomeric products; and/or
wherein the total acetate concentration arising from the GLP2 analogue in the formulation is less than or equal to 11% acetate per mg GLP-2 analogue; and/or
wherein the GLP-2 analogue in the formulation retains at least 90% of its biological activity after 18 months of storage 2-8°C.

**3.** The formulation according to claim 1 or claim 2, wherein formation of covalently linked oligomers of the GLP-2 analogue is inversely dependent on the concentration of the GLP-2 analogue in the formulation.

**4.** The formulation according to any one of claims 1 to 3, wherein the formulation is an aqueous formulation.

**5.** The formulation according to any one of the preceding claims, wherein the formulation has a viscosity between 0.8 and 2.0 mPa/sec measured at 25°C.

**6.** The formulation according to any one of the preceding claims, wherein the GLP-2 analogue is present in the formulation at a concentration of about 15 mg/mL to about 25 mg/ml.

**7.** The formulation according to any one of claims 1 to 5, wherein the GLP-2 analogue is present in the formulation at a concentration of about 20 mg/mL, or at a concentration of about 10 mg/mL, or at a concentration of about 5 mg/mL, or at a concentration of about 2 mg/mL.

**8.** The formulation according to any one of the preceding claims, wherein the formulation consists of the GLP-2 analogue at a concentration of about 2 mg/mL to about 30 mg/ml, a buffer selected from the group consisting of a histidine buffer, mesylate buffer and acetate buffer, the buffer being present at a concentration of about 5 mM to about 50 mM, a non-ionic tonicity modifier selected from the group consisting of mannitol, sucrose, glycerol and sorbitol at a concentration of about 90 mM to about 360 mM, arginine q.s. to provide a pH of about 6.6 to about 7.4.

**9.** The formulation according to any one of the preceding claims, wherein the formulation comprises the GLP-2 analogue at a concentration of about 20 mg/mL, histidine buffer at a concentration of about 15 mM, mannitol at a concentration of about 230 mM, and arginine q.s. to provide a pH of about 7.0.

**10.** The formulation according to any one of the preceding claims, wherein the histidine buffer is present at a concentration of about 5 mM to about 25 mM, optionally wherein the histidine buffer is present at a concentration of about 15 mM.

**11.** The formulation according to any one of the preceding claims, wherein the mannitol is present at a concentration of about 150 mM to about 250 mM, optionally wherein the mannitol is present at a concentration of about 230 mM.

**12.** A method for modulating the viscosity of a stable liquid pharmaceutical formulation comprising a glucagon-like peptide 2 (GLP-2) analogue, wherein the GLP-2 analogue is represented by the formula:

$R^1$-$Z^1$-His-Gly-Glu-Gly-X5-Phe-Ser-Ser-Glu-Leu-X11-Thr-Ile-Leu-Asp-Ala-Leu-

Ala-Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the method comprises formulating (a) the GLP-2 analogue at a concentration of about 2 mg/mL to about 30 mg/mL, (b) with a buffer selected from the group consisting of a histidine buffer, mesylate buffer, acetate buffer, glycine buffer, lysine buffer, TRIS buffer, Bis-Tris buffer or MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the total acetate concentration arising from the GLP2 analogue in the formulation is less than or equal to 11% acetate per mg GLP-2 analogue and wherein the formulation has a viscosity greater than 0.8 and lower than or equal to 2.0 mPa/sec measured at 25°C.

**13.** A method for reducing the formation of covalently bonded oligomeric products of a glucagon-like peptide 2 (GLP-2) analogue in a stable liquid pharmaceutical formulation comprising a GLP-2 analogue represented by the formula:

$R^1$-$Z^1$-His-Gly-Glu-Gly-X5-Phe-Ser-Ser-Glu-Leu-X11-Thr-Ile-Leu-Asp-Ala-Leu-

Ala-Ala-Arg-Asp-Phe-Ile-Ala-Trp-Leu-Ile-Ala-Thr-Lys-Ile-Thr-Asp-$Z^2$-$R^2$

wherein:

$R^1$ is hydrogen, $C_{1-4}$ alkyl (e.g. methyl), acetyl, formyl, benzoyl or trifluoroacetyl
X5 is Ser or Thr
X11 is Ala or Ser
$R^2$ is $NH_2$ or OH;
$Z^1$ and $Z^2$ are independently absent or a peptide sequence of 1-6 amino acid units of Lys;
or a pharmaceutically acceptable salt or derivative thereof;
wherein the method comprises formulating (a) the GLP-2 analogue at a concentration of about 2 mg/mL to about 30 mg/mL, (b) with a buffer selected from the group consisting of a histidine buffer, mesylate buffer, acetate buffer, glycine buffer, lysine buffer, TRIS buffer, Bis-Tris buffer or MOPS buffer, the buffer being present at a concentration of about 5 mM to about 50 mM; (c) with a non-ionic tonicity modifier selected from the group consisting of mannitol, sucrose, glycerol, sorbitol and trehalose, the non-ionic tonicity modifier being present at a concentration of about 90 mM to about 360 mM; and (d) with arginine q.s. to provide a formulation having a pH of about 6.6 to about 7.4;
wherein the formulation contains 5% or less of the GLP-2 analogue in the form of covalently bonded oligomeric products; and
wherein the total acetate concentration arising from the GLP2 analogue in the formulation is less than or equal to 11% acetate per mg GLP-2 analogue and wherein the formulation has a viscosity greater than 0.8 and lower than or equal to 2.0 mPa/sec measured at 25°C.

**14.** The method according to claim 13, wherein formation of covalently linked oligomers of the GLP-2 analogue is inversely dependent on the concentration of the GLP-2 analogue in the formulation.

**15.** The method of any one of claims 12 to 14, wherein (a) comprises formulating the GLP-2 analogue at a

concentration of about 2 mg/mL, about 5 mg/mL, about 10 mg/mL or about 20 mg/mL.

Figure 1

mAU
UV_VIS_1 WVL:215 nm
min
Oligomers
Monomer
15.0
14.0
13.0
12.0
11.0
10.0
9.0
8.0
7.0
6.0
5.0
4.0
3.0
2.0
1.0
0.0
4.15
4.20
4.30
4.40
4.50
4.60
4.70
4.80
4.90
5.00
5.10
5.20
5.30
5.40
5.50
5.60
5.71

Figure 2

Viscosity [mPa/s]
z-average [nm]
Acetate [%]
Viscosity [mP/s]
z-average [nm]
0 1 2 3 4 5 6 7 8 9 10
0 10 20 30 40 50 60
6 7 8 9 10 11 12 13 14 15

Figure 3

Stability, normalized area%, 20 mg/mL
Normalized Area%
100
99
98
97
96
95
94
93
92
91
90
0
1
2
3
Time (weeks)
Histidine
Glycine
Lysine
TRIS
Bis-TRIS
MOPS
Succinic Acid
MES
Mesylate

Stability, normalized area%, 2 mg/mL
Normalized Area%
99
97
95
93
91
89
0
1
2
3
Time (weeks)
Histidine
Glycine
Lysine
TRIS
Bis-TRIS
MOPS
Succinic Acid
MES
Mesylate

Figure 4

Figure 5

Stability, normalized area%, 20 mg/mL
Normalised Area %
99
97
95
93
91
89
87
85
0
1
2
3
Time (weeks)
Mannitol 20mgmL
Sucrose 20 mgmL
Glycerol 20 mgmL
Sorbitol 20 mgmL
Trehalose 20mgmL

Figure 6

Stability, normalised area%, 2 mg/mL
Normalised Area %
100
95
90
85
80
75
70
0
1
2
3
Time (weeks)
Mannitol 2 mgmL
Sucrose 2 mgmL
Glycerol 2 mgmL
Sorbitol 2 mgmL
Trehalose 2mgmL

Figure 7

Normalised Area %
100
98
96
94
92
90
88
86
84
82
80
0
1
2
3
4
Time / Weeks
Formulation 1
Formulation 2
Formulation 3
Formulation 4
Formulation 5

Figure 8

Normalised Area %
104
102
100
98
96
94
92
90
0
2
4
8
13
Time (Weeks)
No Preservative
Benzalkonium chloride
Methylparaben
Potasium sorbate

Figure 9

Normalized Area%
105.0
100.0
95.0
90.0
85.0
80.0
75.0
0
½
1
3
6
Time / Months
Unpreserved
m-cresol
Phenol

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5789379 A
- WO 9739031 A
- US 6184201 B
- WO 02066511 A
- WO 0141779 A
- WO 2001049314 A
- WO 2006117565 A
- WO 0104156 A

### Non-patent literature cited in the description

- **DRUCKER et al.** *Proc. Natl. Acad. Sci. USA*, 1996, vol. 93, 7911-7916
- **WOJDEMANN et al.** *J. Clin. Endocrinol. Metab*, 1999, vol. 84, 2513-2517
- **BENJAMIN et al.** *Gut*, 2000, vol. 47, 112-119
- **CHEESEMAN**. *Am. J. Physiol.*, 1997, R1965-71
- **GUAN et al.** *Gastroenterology*, 2003, vol. 125, 136-147
- Peptide and Protein Drug Delivery. Marcel Dekker, Inc, 1991, 247-301
- **JONES, A.** *Adv. Drug Delivery Rev*, 1993, vol. 10, 29-90
- Remington's Pharmaceutical Sciences. Mark Publishing Company, 1985
- **KEEFE et al.** *Gut*, 2000, vol. 47, 632-7
- **GIBSON et al.** *J Gastroenterol. Hepatol.*, 2003, vol. 18 (9), 1095-1100
- **TAMAKI et al.** *J. Int. Med. Res.*, 2003, vol. 31 (1), 6-16
- **GIBSON et al.** *J. Gastroenterol. Hepatol. Sep*, 2003, vol. 18 (9), 1095-1100
- **TAMAKI et al.** *J Int. Med. Res.*, 2003, vol. 31 (1), 6-16
- **MAARSCHALKERWEERD et al.** *Intrinsically Disord. Proteins*, 2015, vol. 3 (1), e1071302